(19) Europäisches Patentamt
European Patent Office
Office européen des brevets

(11) Veröffentlichungsnummer: **0 276 194**
**A1**

(12) # EUROPÄISCHE PATENTANMELDUNG

(21) Anmeldenummer: **88810004.7**

(22) Anmeldetag: **06.01.88**

(51) Int. Cl.[4]: **C 07 D 281/02**
C 07 D 417/12, A 61 K 31/55

(30) Priorität: **12.01.87 US 2074**

(43) Veröffentlichungstag der Anmeldung:
**27.07.88 Patentblatt 88/30**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Wasley, Jan W.F.**
**55 Floral Street**
**Chatham New Jersey 07928 (US)**

(54) **2,1-Benzothiazepin-2,2-dioxid-5-carbonsäurederivate.**

(57) Die Erfindung betrifft Verbindungen der Formel I,

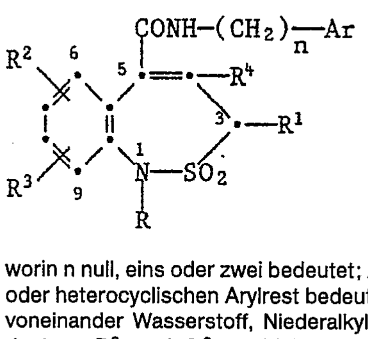

(I)

worin n null, eins oder zwei bedeutet; Ar einen carbocyclischen oder heterocyclischen Arylrest bedeutet; R und $R^1$ unabhängig voneinander Wasserstoff, Niederalkyl oder Ar-Niederalkyl bedeuten; $R^2$ und $R^3$ unabhängig voneinander Wasserstoff, Niederalkyl, Halogen, Trifluormethyl oder Niederalkoxy bedeuten; oder $R^2$ und $R^3$ an benachbarten Kohlenstoffatomen gemeinsam Niederalkylendioxy bedeuten; $R^4$ Hydroxy oder $NR^5R^6$ bedeutet, worin $R^5$ und $R^6$ unabhängig voneinander für Wasserstoff, Niederalkyl oder Ar-Niederalkyl stehen; oder worin $R^5$ und $R^6$ zusammen mit dem Stickstoffatom, an welches sie gebunden sind, für Pyrrolidin oder Piperidin stehen; pharmazeutisch annehmbare Salze davon; und Niederalkylenolether und Niederalkanoylenolester von Verbindungen der Formel I, worin $R^4$ Hydroxy bedeutet, ferner Verfahren zu deren Herstellung, pharmazeutische Präparate, die diese Verbindungen enthalten, sowie Verfahren zur Behandlung beispielsweise von Entzündungszuständen, durch Verabreichung dieser Verbindungen und pharmazeutischer Präparate an Säuger, die einer solchen Verabreichung bedürfen.

EP 0 276 194 A1

**Beschreibung**

2,1-Benzothiazepin-2,2-dioxid-5-carbonsäurederivate

Die vorliegende Erfindung betrifft 1,3-Dihydro-2,1-benzothiazepin-2,2-dioxide, die in Säugern als Inhibitoren der chemotaktischen Aktivierung von neutrophilen Granulozyten, Inhibitoren des Lipoxygenase-Enzymsystems und Inhibitoren des Abbaus von Knorpelgrundsubstanz verwendet werden können. Die vorstehenden Eigenschaften lassen die Verbindungen der vorliegenden Erfindung insbesondere als eine neue Klasse therapeutischer Wirkstoffe nützlich erscheinen zur Behandlung von Entzündungszuständen wie rheumatischer Arthritis in Säugern. Die erfindungsgemässen Verbindungen können darüber hinaus auch bei der Behandlung von Asthma, Allergien, Osteoarthritis und Herzischämie bei Säugern verwendet werden.

Die Erfindung bezieht sich insbesondere auf die nachstehende beschriebenen Verbindungen, Verfahren zu deren Herstellung, pharmazeutische Präparate, die diese Verbindungen enthalten, Verfahren zur Behandlung beispielsweise von Entzündungszuständen, durch Verabreichung dieser Verbindungen und pharmazeutischer Präparate an Säuger, die einer solchen Verabreichung bedürfen.

Insbesondere bezieht sich die Erfindnung auf die Verbindungen der Formel I,

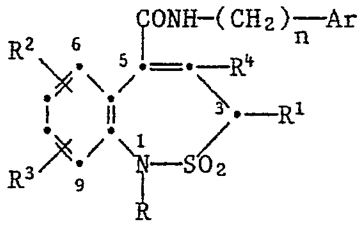

(I)

worin n null, eins oder zwei bedeutet; Ar einen carbocyclischen oder heterocyclischen Arylrest bedeutet; R und $R^1$ unabhängig voneinander Wasserstoff, Niederalkyl oder Ar-Niederalkyl bedeuten; $R^2$ und $R^3$ unabhängig voneinander Wasserstoff, Niederalkyl, Halogen, Trifluoromethyl oder Niederalkoxy bedeuten; oder $R^2$ und $R^3$ an benachbarten Kohlenstoffatomen gemeinsam Niederalkylendioxy bedeuten; $R^4$ Hydroxy oder $NR^5R^6$ bedeutet, worin $R^5$ und $R^6$ unabhängig voneinander für Wasserstoff, Niederalkyl oder Ar-Niederalkyl stehen; oder worin $R^5$ und $R^6$ zusammen mit dem Stickstoffatom, an welches sie gebunden sind, für Pyrrolidin oder Piperidin stehen; pharmazeutisch annehmbare Salze davon; und Niederalkylenolether und Niederalkanoylenolester von Verbindungen der Formel I, worin $R^4$ Hydroxy bedeutet.

Bestimmte Verbindungen der Formel I können auch als Tautomere vorliegen, die von dieser Erfindung mitumfasst sind.

Tautomere der Verbindungen der Formel I, worin $R^4$ Hydroxy oder $NHR^5$ bedeutet, entsprechen den 4-Oxo- oder 4-Iminotautomeren, d.h. den 5-Carbamoyl-substituierten 1,3,4,5-Tetrahydro-4-oxo-2,1-benzothiazepin-2,2-dioxiden, welche durch die Formel Ia

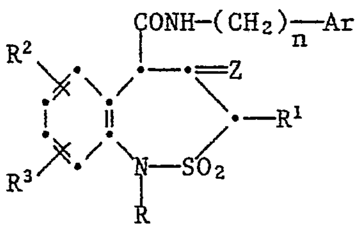

(Ia)

wiedergegeben werden, worin n, Ar, R, $R^1$, $R^2$ und $R^3$ die vorstehend angegebenen Bedeutungen aufweisen; und worin Z Sauerstoff (O) oder $NR^5$ bedeutet, worin $R^5$ für Wasserstoff, Niederalkyl oder Ar-Niederalkyl steht.

Insbesondere bezieht sich die Erfindung auf Verbindungen der Formel I, worin Ar für Phenyl steht oder für Phenyl, welches mono-oder disubstituiert ist durch einen Substituenten ausgewählt aus der Gruppe bestehend aus Niederalkyl, Niederalkoxy, Niederalkylthio, Niederalkylsulfinyl, Niederalkylsulfonyl, Halogen, Cyan, Carboxy, Niederalkoxycarbonyl, Carbamoyl und Trifluormethyl; oder worin Ar 1-oder 2-Naphthyl bedeutet; oder worin Ar einen fünfgliedrigen ungesättigten heterocyclischen Rest bedeutet, der über Kohlenstoff gebunden ist und ein Heteroatom ausgewählt unter Schwefel, Sauerstoff und unsubstituiertem oder durch Niederalkyl substituiertem Aminostickstoff enthält, oder einen solchen Rest, der zwei Heteroatome, ausgewählt unter einem Iminostickstoff und einem Vertreter aus der Gruppe unsubstituierter oder durch Niederalkyl substituierter Aminostickstoff, Schwefel und Sauerstoff, enthält; oder worin Ar einen sechsgliedrigen ungesättigten heterocyclischen Rest bedeutet, der über Kohlenstoff gebunden ist und ein oder zwei Stickstoffatome enthält; oder worin Ar einen bicyclischen benzokondensierten fünfgliedrigen ungesättigten heterocyclischen Rest bedeutet, der über Kohlenstoff gebunden ist und ein Heteroatom, ausgewählt unter Schwefel, Sauerstoff und unsubstituiertem oder durch Niederalkyl substituiertem Aminostickstoff, enthält; oder worin Ar einen bicyclischen benzokondensierten fünfgliedrigen ungesättigten

heterocyclischen Rest bedeutet, der über Kohlenstoff gebunden ist und zwei Heteroatome, ausgewählt unter einem Iminostickstoff und einem Vertreter aus der Gruppe unsubstituierter oder durch Niederalkyl substituierter Aminostickstoff, Sauerstoff und Schwefel, enthält; oder worin Ar einen bicyclischen benzokondensierten sechsgliedrigen ungesättigten heterocyclischen Rest bedeutet, der über Kohlenstoff gebunden ist und ein oder zwei Stickstoffatome enthält; oder worin Ar einen dieser heterocyclischen Reste bedeutet, die an Kohlenstoffatomen durch Niederalkoxy, Niederalkyl oder Halogen mono- oder disubstituiert sind; worin n, R und $R^1$-$R^6$ wie vorstehend definiert sind; pharmazeutisch annehmbare Salze davon; die entsprechenden Tautomere der Formel Ia von Ver bindungen der Formel I, worin $R^4$ für Hydroxy oder $NHR^5$ steht; Niederalkylenolether davon und Niederalkanoylenolester von Verbindungen, worin $R^4$ Hydroxy bedeutet.

Bevorzugt sind die Verbindungen der Formel I, worin Ar Phenyl bedeutet oder Phenyl, welches mono- oder disubstituiert ist durch einen Rest ausgewählt aus der Gruppe bestehend aus Niederalkyl, Niederalkoxy, Niederalkylthio, Niederalkylsulfinyl, Niederalkylsulfonyl, Halogen, Cyan, Carboxy, Nideralkoxycarbonyl, Carbamoyl und Trifluormethyl; oder worin Ar einen fünfgliedrigen ungesättigten heterocyclischen Rest bedeutet, der über Kohlenstoff gebunden ist und zwei Heteroatome, ausgewählt unter einem Iminostickstoff und einem Vertreter aus der Gruppe unsubstituierter oder durch Niederalkyl substituierter Aminostickstoff, Schwefel und Sauerstoff, enthält; oder worin Ar einen sechsgliedrigen ungesättigten heterocyclischen Rest bedeutet, der über Kohlenstoff gebunden ist und ein oder zwei Stickstoffatome enthält; worin n, R und $R^1$-$R^6$ wie vorstehend definiert sind; pharmazeutisch annehmbare Salze davon; die entsprechenden Tautomere der Formel Ia von Verbindungen der Formel I, worin $R^4$ für Hydroxy oder $NHR^5$ steht.

Bevorzugt sind die Verbindungen der Erfindung, worin n null bedeutet.

Weiterhin bevorzugt sind die Verbindungen der Formel I oder Tautomere davon, worin Ar Phenyl bedeutet oder Phenyl, welches mono- oder disubstituiert ist durch einen Rest ausgewählt aus der Gruppe bestehend aus Niederalkyl, Niederalkoxy, Niederalkylthio, Niederalkylsulfinyl, Niederalkylsulfonyl, Halogen, Cyan, Carboxy, Niederalkoxycarbonyl, Carbamyol und Trifluoromethl; oder worin Ar 1- oder 2-Napthyl bedeutet; oder worin Ar einen heterocyclischen Rest bedeutet, der über Kohlenstoff an das Amid-Stickstoffatom gebunden ist und augewählt ist unter Furyl, Pyrrolyl, Thienyl, Thiazolyl, Oxazolyl, Pyridyl, Pyrimidyl, Pyrazinyl, Benzofuranyl, Indolyl, Isochinolyl, Chinolyl, Imidazolyl, Isoxazolyl, Benzimidazolyl, Benzoxazolyl oder einem dieser heterocyclischen Reste, die an Kohlenstoff durch Niederalkyl, Niederalkoxy oder Halogen mono- oder disubstituiert sind, oder einem dieser unsubstituierten oder substituierten Pyrrolyl-, Indolyl-, Imidazolyl- oder Benzimidazolyl-Reste, die am Stickstoffatom durch Niederalkyl substituiert sind; worin n null bedeutet; $R^4$ Hydroxy bedeutet; R, $R^1$, $R^2$ und $R^3$ wie vorstehend definiert sind; pharmazeutisch annehmbare Salze davon; Niederalkylenolether davon; und Niederalkanoylenolester davon.

Besonders bevorzugt sind die Verbindungen der Formel I oder Tautomere davon, worin n null bedeutet; Ar für Phenyl steht oder für Phenyl, welches mono- oder disubstituiert ist durch enen Rest ausgewählt aus der Gruppe bestehend aus Niederalkyl, Niederalkoxy, Niederalkylthio, Niederalkylsulfinyl, Niederlakylsulfonyl, Halogen und Trifluormethyl; oder worin Ar einen heterocyclischen Rest bedeutet, der über Kohlenstoff an das Amid-Stickstoffatom gebunden ist und ausgewählt ist unter Furyl, Pyrrolyl, Thienyl, Thiazolyl, Oxazolyl, Pyridyl, Pyrimidyl, Pyrazinyl, Benzofuranyl, Indolyl, Isochinolyl und Chinolyl, oder einem dieser heterocyclischen Reste, die am Kohlenstoff durch Niederalkyl, Niederalkoxy oder Halogen mono- oder disubstituiert sind, oder einem unsubstituierten oder substituierten Pyrrolyl- oder Indolyl-Rest, welcher am Stickstoff durch Niederalkyl substituiert ist; worin R und $R^1$ unabhängig voneinander Wasserstoff, Niederalkyl, Phenylniederalkyl oder Phenylniederalkyl, welches am Phenylteil durch einen oder zwei Reste, ausgewählt unter Niederalkyl, Niederalkoxy, Niederalkylthio, Niederalkylsulfinyl, Niederalkylsulfonyl, Halogen und Trifluormethyl, substituiert ist, bedeuten; $R^2$ und $R^3$ unabhängig voneinander Wasserstoff, Niederalkyl, Halogen, Trifluoromethyl oder Niederalkoxy bedeuten; $R^4$ für Hydroxy steht; pharmazeutisch annehmbare Salze davon, die von pharmazeutisch annehmbaren Basen abgeleitet sind; oder pharmazeutisch annehmbare Säureadditionssalze davon, falls Ar einen basischen heterocyclischen Rest bedeutet; Niederalkylenoletherderivate davon; sowie Niederalkanoylenolesterderivate davon.

Ebenfalls bevorzugt sind die Verbindungen der Formel I oder Tautomere davon, worin n null, eins oder zwei bedeutet; Ar für Phenyl steht oder Phenyl bedeutet, welches mono- oder disubstituiert ist durch einen Rest ausgewählt aus der Gruppe bestehend aus Niederalkyl, Niederalkoxy, Niederalkylthio, Carboxy, Halogen und Trifluoromethyl; oder worin Ar Thiazolyl odr Pyridyl bedeutet; worin R und $R^1$ unabhängig voneinander Wasserstoff, Niederalkyl, Phenylniederalkyl oder Phenylniederalkyl, welches im Phenylteil durch einen oder zwei Reste, ausgewählt unter Niederalkyl, Niederalkoxy, Nideralkyltio, Halogen und Trifluormethyl, substituiert ist, bedeuten; $R^2$ und $R^3$ unabhängig voneinander Wasserstoff, niederalkyl, Halogen, Trifluoromethyl oder Niederalkoxy bedeuten; $R^4$ für Hydroxy, Pyrrolidino, Phenylniederalkylamino oder Phenylniederalkylamino, welches im Phenylteil durch Halogen substituiert ist, steht; pharmazeutisch annehmbare Salze davon, die von pharmazeutisch annehmbaren Basen abgeleitet sind; oder pharmazeutisch annehmbare Säureadditionssalze davon, falls Ar einen basischen heterocyclischen Rest bedeutet; Niederalkylenoletherderivate davon; und Niederalkanoylenolesterderivate davon.

Weiterhin bevorzugt sind Verbindungen der Formel I oder Tautomere davon, worin n null bedeutet, Ar für Phenyl steht oder für Phenyl, welches durch $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, Halogen oder Trifluormethyl monosubstituiert ist, oder für Phenyl, welches durch Reste ausgewählt unter $C_1$-$C_4$-Alkyl, $C_1$-$C_4$-Alkoxy und Halogen disubstituiert ist; worin R Wasserstoff, $C_1$-$C_4$-Alkyl,

Phenyl-$C_1$-$C_4$-alkyl oder Phenyl-$C_1$-$C_4$-alkyl, welches im Phenylteil durch $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, Halogen oder Trifluormethyl monosubstituiert ist, oder Phenyl-$C_1$-$C_4$-Alkyl, welches im Phenylteil durch Reste ausgewählt unter $C_1$-$C_4$-Alkyl, $C_1$-$C_4$-Alkoxy und Halogen disubstituiert ist, bedeutet; worin $R^1$ Wasserstoff, $C_1$-$C_4$-Alkyl, Phenyl-$C_1$-$C_4$-alkyl oder Phenyl-$C_1$-$C_4$-alkyl, welches im Phenylteil durch $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, Halogen oder Trifluormethyl monosubstituiert ist, oder Phenyl-$C_1$-$C_4$-alkyl, welches im Phenylteil durch Reste ausgewählt unter $C_1$-$C_4$-Alkyl, $C_1$-$C_4$-Alkoxy und Halogen disubstituiert ist, bedeutet; worin $R^2$ Wasserstoff, $C_1$-$C_4$-Alkyl, $C_1$-$C_4$-Alkoxy, Halogen oder Trifluormethyl bedeutet; $R^3$ für Wasserstoff, $C_1$-$C_4$-Alkyl, $C_1$-$C_4$-Alkoxy oder Halogen steht; $R^4$ Hydroxy bedeutet; und pharmazeutisch annehmbare Salze abgeleitet von pharmazeutisch annehmbaren Basen.

Ebenfalls bevorzugt sind Verbindungen der Formel I oder Tautomere davon, worin n null bedeutet; Ar einen heterocyclischen Rest bedeutet ausgewählt unter 2-Pyridyl und 2-Thiazolyl, oder einen solchen Rest, der an Kohlenstoff durch $C_1$-$C_4$-Alkyl oder Halogen substituiert ist; R Wasserstoff, $C_1$-$C_4$-Alkyl, Phenyl-$C_1$-$C_4$-alkyl oder Phenyl-$C_1$-$C_4$-alkyl, welches im Phenylteil durch $C_1$-$C_4$-Alkyl, $C_1$-$C_4$-Alkoxy, $C_1$-$C_4$-Alkylthio, Halogen oder Trifluormethyl monosubstituiert ist, oder Phenyl-$C_1$-$C_4$-alkyl, welches im Phenylteil durch einen Rest ausgewählt unter $C_1$-$C_4$-Alkyl, $C_1$-$C_4$-Alkoxy und Halogen disubstituiert ist, bedeutet; $R^1$ für Wasserstoff, $C_1$-$C_4$-Alkyl, Phenyl-$C_1$-$C_4$-alkyl oder Phenyl-$C_1$-$C_4$-alkyl, welches im Phenylteil durch $C_1$-$C_4$-Alkyl, $C_1$-$C_4$-Alkoxy, $C_1$-$C_4$-Alkylthio, Halogen oder Trifluormethyl monosubstituiert ist, oder Phenyl-$C_1$-$C_4$-alkyl, welches im Phenylteil durch einen Rest ausgewählt unter $C_1$-$C_4$-Alkyl, $C_1$-$C_4$-Alkoxy und Halogen disubstituiert ist, bedeutet; worin $R^2$ Wasserstoff, $C_1$-$C_4$-Alkyl, $C_1$-$C_4$-Alkoxy, Halogen oder Trifluormethyl bedeutet; $R^3$ für Wasserstoff, $C_1$-$C_4$-Alkyl, $C_1$-$C_4$-Alkoxy oder Halogen steht; $R^4$ Hydroxy bedeutet; und pharmazeutisch annehmbare Salze davon, abgeleitet von pharmazeutisch annehmbaren Basen, oder pharmazeutisch annehmbare Säureadditionssalze davon.

Weiterhin bevorzugt sind die Verbindungen der Formel II,

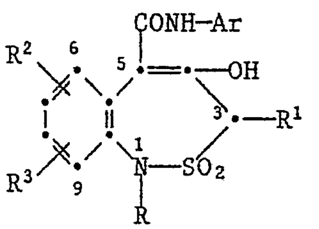

(II)

oder Tautomere davon, worin Ar für Phenyl steht oder Phenyl bedeutet, welches durch $C_1$-$C_4$-Alkyl, $C_1$-$C_4$-Alkoxy, $C_1$-$C_4$-Alkylthio, Halogen oder Trifluormethyl monosubstituiert ist; R $C_1$-$C_4$-Alkyl, Phenyl-$C_1$-$C_4$-alkyl oder Phenyl-$C_1$-$C_4$-alkyl, welches im Phenylteil durch Halogen oder Trifluormethyl monosubstituiert ist, bedeutet; $R^1$ für Wasserstoff, $C_1$-$C_4$-Alkyl oder Phenyl-$C_1$-$C_4$-alkyl steht; $R^2$ Wasserstoff, Halogen, Trifluormethyl oder $C_1$-$C_4$-Alkyl bedeutet; $R^3$ Wasserstoff bedeutet; und pharmazeutisch annehmbare Salze abgeleitet von pharmazeutisch annehmbaren Basen.

Insbesondere bevorzugt sind die Verbindungen der Formel II oder Tautomere davon, worin Ar Phenyl bedeutet oder Phenyl, welches durch Methyl, Methylthio, Methoxy, Chlor, Fluor oder Trifluormethyl monosubstituiert ist, R Methyl, Benzyl oder Benzyl, welches im Phenylteil durch Halogen oder Trifluormethyl substituiert ist, bedeutet; $R^1$, $R^2$ und $R^3$ Wasserstoff bedeutet; und pharmazeutisch annehmbare Salze abgeleitet von pharmazeutisch annehmbaren Basen.

Die allgemeinen Definitionen, die hier verwendet werden, haben im Zusammenhang mit der vorliegenden Erfindung die folgenden Bedeutungen:

Der Ausdruck "Nieder", der vorstehend und nachstehend im Zusammenhang mit organischen Resten oder Verbindungen verwendet wird, definiert solche mit bis zu und einschliesslich 7, vorzugsweise bis zu und einschliesslich 4 und vorzugsweise einem oder zwei Kohlenstoffatomen.

Niederalkyl enthält vorzugsweise 1 - 4 Kohlenstoffatome und bedeutet z.B. Ethyl, Propyl, Butyl oder vorzugsweise Methyl.

Niederalkoxy enthält vorzugsweise 1 - 4 Kohlenstoffatome und bedeutet z.B. Ethoxy, Propoxy, Isopropoxy oder vorteilhafterweise Methoxy; Niederalkylthio enthält vorzugsweise 1 - 4 Kohlenstoffatome und bedeutet vorteilhafterweise Methylthio oder Ethylthio; Niederalkylsulfinyl enthält vorzugsweise 1 - 4 Kohlenstoffatome und bedeutet vorteilhafterweise Methylsulfinyl oder Ethylsulfinyl; Niederalkylsulfonyl enthält vorzugsweise 1 - 4 Kohlenstoffatome und bedeutet vorteilhafterweise Methylsulfonyl oder Ethylsulfonyl.

Niederalkoxycarbonyl enthält vorzugsweise 1 - 4 Kohlenstoffatome im Alkoxyteil und bedeutet beispielsweise Methoxycarbonyl oder Ethoxycarbonyl.

Niederalkylendioxy enthält vorzugsweise 1 - 4 Kohlenstoffatome und bedeutet beispielsweise Methylendioxy oder Ethylendioxy.

Halogen bedeutet vorzugsweise Chlor oder Fluor, kann jedoch auch Brom oder lod sein.

Ein Arylrest (Ar) bedeutet einen carbocyclischen oder einen heterocyclischen Arylrest.

Ein carbocyclischer Arylrest bedeutet vorzygsweise Phenyl oder Phenyl, welches mono- oder disubstituiert ist durch einen Rest ausgewählt aus der Gruppe bestehend aus Niederalkyl, Niederalkoxy, Niederalkylthio,

Niederalkylsulfinyl, Niederalkylsulfonyl, Halogen, Cyan, Carboxy, Niederalkoxycarbonyl, Carbamoyl und Trifluormethyl; ferner 1- oder 2-Naphthyl.

Ein heterocyclischer Arylrest, insbesondere Ar in der Einheit CONH$(CH_2)_n$-Ar, ist vorzugsweise über Kohlenstoff an den CONH$(CH_2)_n$-Rest gebunden, d.h. an ein $CH_2$-Kohlenstoffatom, falls n 1 oder 2 bedeutet, oder an das Amid-Stickstoffatom, falls n null bedeutet.

Ein heterocyclischer Arylrest bedeutet vorzygsweise einen aromatischen Rest wie beispielsweise Furyl, Pyrrolyl, Thienyl, Thiazolyl, Oxazolyl, Pyridyl, Pyrimidyl, Pyrazinyl, Benzofuranyl, Indolyl, Isochinolyl oder Chinolyl, kann aber auch Imidazolyl, Isoxazolyl, Benzimidazolyl, Benzoxazolyl oder einen dieser heterocyclischen Reste, die am Kohlenstoff durch Niederalkyl, Niederalkoxy oder Halogen mono- oder disubstituiert sind, bedeuten, oder einen unsubstituierten oder so substituierten Pyrrolyl, Indolyl-, Imidazolyl- oder Benzimidazolyl-Rest, der zusätzlich am Stickstoff durch Niederalkyl substituiert ist.

Furyl bedeutet vorzugsweise 2-Furyl. Pyrrolyl bedeutet vorzugsweise 2-Pyrrolyl. Thienyl bedeutet vorzugsweise 2-Thienyl. Thiazolyl bedeutet vorzugsweise 2-Thiazolyl. Oxazolyl bedeutet vorzugsweise 2-Oxazolyl. Pyridyl bedeutet vorzugsweise 2- oder 4-Pyridyl, vorteilhafterweise 2-Pyridyl. Pyrimidyl bedeutet vorzugsweise 2-Pyrimidyl. Pyrazinyl bedeutet vorzugsweise 2-Pyrazinyl. Benzofuranyl bedeutet vorzugsweise 2-Benzofuranyl. Indolyl bedeutet vorzugsweise 2-Indolyl. Isochinolyl bedeutet vorzugsweise 4-Isochinolyl. Chinolyl bedeutet vorzugsweise 4-Chinolyl.

Niederalkanoyl bedeutet vorzugsweise $C_1$-$C_4$-Alkanoyl, z.B. Acetyl oder Propionyl.

Die Verbindungen der Formel I, worin $R^4$ Hydroxy bedeutet, haben saure Eigenschaften und bilden Niederalkylenolether, Niederalkanoylenolester, oder Salze davon.

Pharmazeutisch annehmbare Salze werden mit pharmazeutisch annehmbaren Basen gebildet, wie mit Alkalimetall-, Erdalkalimetall-, Kupfer- oder Zinkhydroxiden, Ammoniak, mono-, di- oder trinieder-(Alkyl oder Hydroxyalkyl)-Aminen, Trihydroxyniederalkylaminen, monocyclischen Aminen oder Alkylendiaminen, und sind z.B. die Natrium-, Kalium-, Magnesium-, Ammonium-, mono-, di- oder tri-(Methyl, Ethyl oder Hydroxyethyl)-Ammonium-, Tromethamin-, Pyrrolidinium-, Ethylendiammonium- oder Morpholiniumsalze.

Pharmazeutische annehmbare Salze von Verbindungen der Erfindung, die eine basische Gruppe tragen, sind Säureadditionssalze, vorzugsweise solche von therapeutisch annehmbaren anorganischen oder organischen Säuren, wie starken Mineralsäuren, beispielsweise Halogenwasserstoffsäuren, z.B. Chlorwasserstoff oder Bromwasserstoffsäure, Schwefelsäure, Phosphorsäure oder Salpetersäure; aliphatischen oder aromatischen Carbonsäuren oder Sulfonsäuren, z.B. Ameisensäure, Essigsäure, Propionsäure, Bernsteinsäure, Glykolsäure, Milchsäure, Aepfelsäure, Weinsäure, Glukonsäure, Zitronensäure, Maleinsäure, Fumarsäure, Brenztraubensäure, Phenylessigsäure, Benzoesäure, 4-Aminobenzoesäure, Anthranilsäure, 4-Hydroxybenzoesäure, Salicylsäure, 4-Aminosalicylsäure, Pamoasäure, Nikotinsäure, Methansulfonsäure, Ethansulfonsäure, Hydroxyethanseulfonsäure, Benzolsulfonsäure, p-Toluolsulfonsäure, Naphthalinsulfonsäure, Sulfanilsäure, Cyclohexylsulfamsäure; oder Ascorbinsäure.

Die erfindungsgemässen Verbindungen weisen wertvolle pharmakologische Eigenschaften in Säugern auf, insbesondere als Inhibitoren der chemotaktischen Aktivierung von neutrophilen Granulozyten; sie sind auch aktiv als selektive 5-Lipoxygenasehemmer und als Inhibitoren des Abbaus von Knorpelgrundsubstanz.

Die erfindungsgemässen Verbindungen sind daher nützlich bei der Behandlung und Heilung von Krankheiten in Säugern, bei denen erhöhte neutrophile Aktivierung, erhöhte Lipoxygenaseaktivität oder erhöhter Knorpelabbau eine Rolle spielen. Neutrophile Aktivierung ist z.B. bei rheumatischer Arthritis beteiligt, 5-Lipoxygenase z.B. bei verschiedenen allergischen Zuständen und Asthma, und Knorpelabbau z.B. bei der Osteoarthritis.

Die genannten Eigenschaften lassen sich in in vitro und in vivo-Versuchen, vorzugsweise bei Säugetieren, z.B. Mäusen, Guineaschweinen, Hunden, Kaninchen, aber auch an entnommenen isolierten Organen, Gewebsproben oder enzymatischen Aufbereitungen davon, wie auch an Zellen und Flüssigkeiten, die aus dem menschlichen Blut abgetrennt wurden, nachweisen. Die Verbindungen können in vitro in Form von Lösungen, z.B. vorzugsweise wässrigen Lösungen, und in vivo entweder enteral oder parenteral, mit Vorteil oral, z.B. als Suspension oder in wässriger Lösung verabreicht werden. Der Dosis bereich kann in vitro zwischen $10^{-4}$ und $10^{-9}$ molaren Konzentrationen betragen. Der Dosisbereich in vivo kann, je nach Art der Verabreichung, zwischen etwa 0,01 und 50 mg/kg, vorteilhafterweise zwischen etwa 0,10 und 25 mg/kg betragen.

Die Inhibierung der chemotaktischen Aktivierung von neutrophilen Granulozyten wird in vitro bestimmt, indem z.B. die Inhibierung der Bindung von f-MLP (Formyl-methionyl-leucyl-phenylalanin) an menschliche neutrophile Granulozyten in vitro wie folgt gemessen wird:

Menschliche neutrophile Granulozyten werden gemäss einer Modifizierung des Verfahrens von Ferrante und Thong (J. Immunol. Methods <u>24</u>, 389, 1978) isoliert. Rote Blutkörperchen, die in der neutrophilen Präparation zurückbleiben, werden durch separate Behandlungen mit 0,83 % Ammoniumchlorid und 0,1 M Tris-HCl, pH 7,5, zerstört. Die neutrophilen Granulozyten werden in Hanks-Puffer bei 0°C während 60 Minuten mit 15 nM $^3$H-f-MLP und Testverbindung inkubiert. Aliquote des Inkubats werden filtriert, und das Total des gebundenen $^3$H-f-MLP wird gemessen. Nichtspezifische Bindung in Anwesenheit von überschüssigem nicht-radioaktivem f-MLP wird abgezogen, und die prozentuale Inhibierung der Bindung durch die Testverbindung wird ermittelt. Die Endkonzentrationen von Lösungsmitteln, wie Dimethylacetamid oder Ethanol, welche verwendet werden können, um die Testverbndung zu lösen, dürfen in der Inkubationsmischung 1 % nicht überschreiten.

Die Inhibierung der chemotaktischen Aktivierung von neutrophilen Granulozyten kann auch in vivo bestimmt

werden, indem die Inhibierung der Akkumulation von neutrophilen Granulozyten in Carragheenimprägnierte Polyurethanschwämme nach oraler Verabreichung in der Ratte gemessen wird.

Lipoxygenaseeinhibierung wird beispielsweise bestimmt, indem der Anteil Inhibierung bei der Synthese von 5-HETE [(5S)-5-Hydroxy-6,8,11,14-eikosatetraencarbonsäure] und Leukotrien $B_4$ ($LTB_4$, 5,12-Dihydroxy-6,8,10,14-eikosatetraencarbonsäure) in A-23187- stimulierten polymorphkernigen Leukozyten von Meerschweinchen gemessen wird, insbesondere gemäss den radiometrischen dünnschichtchromatographischen Tests, die von Walker und Dawson beschrieben sind (J. Pharm. Pharmacol. 31,778, 1979) und von Jakschik und Lee (Nature 287, 51, 1980), welche verwendet werden, um die Bildung von 5-HETE und $LTB_4$-artigen Verbindungen aus $^{14}C$-Arachidonsäure zu messen.

Weiterhin wird die Inhibierung der Bildung von Leukotrienen und 5-HETE z.B. bestimmt, indem ihre Konzentration im gesamten Blut nach oraler Verabreichung in Ratten gemessen wird.

Antiinflammatorische Aktivität wird bestimmt, indem die Inhibierung von Oedemen gemessen wird und des mononuklearen Zellzuflusses nach oraler Verabreichung im Rattenmodell, in dem zuerst Pleuritis induziert wird durch Injektion von Carragheen in die Pleurahöhle, z.B. gemäss A.P. Almeida et al., J. Pharmacol. Exp. Therap. 214, 74 (1980).

Die Inhibierung des Abbaus von Knorpelgrundsubstanz kann in vitro bestimmt werden an Hand der Rinder-Nasenknorpel-Synovium co-culture Modells des Abbaus von Knorpelgrundsubstanz, welches wie folgt ausgeführt wird:

Die Proteoglykanmatrix von Rinder-Nasenknorpel-Schweidewand wird in vitro durch Aufnahme von $^{35}S$ in Glykosaminoglykan markiert. Knorpelstücke werden über Nacht in einem sulfatfreien Medium inkubiert, welches $^{35}S$-Natriumsulfat enthält. $^{35}S$-markierte Knorpelstücke werden gemeinsam kultiviert mit normalem Scheidewand-Explantat auf Multiwellgewebekulturplatten. Nach 4 Tagen Inkubation wird ein 100 Mikroliter Aliquot des Mediums ausgezählt. Knorpelstücke werden hydrolisiert und ein 100 Mikroliter Aliquot des Knorpelhydrolysats wird ausgezählt. Der Anteil an $^{35}S$, der in das Medium hinein freigesetzt wurde, wird bestimmt, und der Prozentsatz Inhibierung des Abbaus von Knorpelgrundsubstanz wird ermittelt. Die Inhibierung des Abbaus von Knorpelgrundsubstanz kann auch auf ähnliche Weise bestimmt werden, indem die Abnahme an Proteoglykanfreisetzung bei dem synovial katabolininduzierter Abbau von Rinder-Nasenknorpel gemessen wird.

Eine representätive erfindungsgemässe Verbindung, als Natriumsalz, ist bei der Inhibierung der Bindung von f-MLP an menschliche neutrophile Granulozyten in einer Konzentration von etwa $1 \times 10^{-6}$ M wirksam. Weiterhin vermindert die Verbindung wirksam die Akkumulierung von neutrophilen Granulozyten in mit Caragheen imprägnierte Schwämme in der Ratte bei einer Dosis von 25 mg/kg p.o., inhibiert wirksam Leukotrien- und 5-HETE-Bildung im gesamten Rattenblut bei einer Dosis von 50 mg/kg p.o., inhibert signifikant Oedembildung und mononuklearen Zellzufluss im Pleuritismodell der Entzündung bei einer Dosis von 10 mg/kg p.o. in der Ratte, und inhibiert bei einer Konzentration von etwa $1 \times 10^{-5}$ M die Freisetzung von Proteoglykan in katabolininduziertem oder synoviuminduziertem Abbau von Knorpel.

Im Gegensatz zu klassischen antiinflammatorischen Wirkstoffen sind die Verbindungen der vorliegenden Erfindung nicht wirksam in den Tests, die normalerweise verwendet werden, um solche Stoffe zu untersuchen, wie in dem Carragheen Pfotenödem Test, dem Phenylchinon-Ventrikeltest und dem etablierten Adjuvantarthritistest.

Die verstehend genannten Eigenschaften lassen die Verbindungen dieser Erfindung nützlich erscheinen insbesondere als Krankheiten beeinflussende antiinflammatorische und antiarthritische Mittel, besonders zur Behandlung und Heilung von Entzündungskrankheiten, wie rheumatischer Arthritis und Osteoarthritis in Säugetieren, unter Einschluss des Menschen.

Die erfindungsgemässen Verbindungen können auf an sich bekannte Weise hergestellt werden, indem man z.B.

a) ein Keton der Formel III,

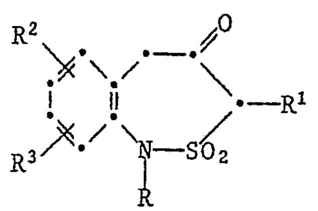

(III)

worin R, $R^1$, $R^2$ und $R^3$ die vorstehend angegebenen Bedeutungen aufweisen, oder ein Enaminderivat der Formel IIIa,

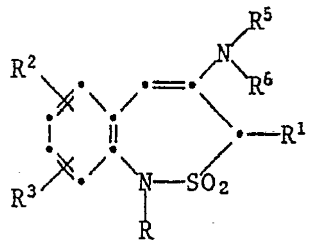

(IIIa)

worin R, $R^1$, $R^2$, $R^3$, und n die vorstehend angegebenen Bedeutungen aufweisen, und $R^5$ und $R^6$ Niederalkyl bedeuten, oder $R^5$ und $R^6$ zusammen mit dem Stickstoffatom, an das sie gebunden sind, Pyrrolidin oder Piperidin bedeuten, mit einer Verbindung der Formel IV,

$Ar\text{-}(CH_2)_n\text{-}N=C=O$ (IV)

worin Ar und n die vorstehend angegebenen Bedeutungen aufweisen, kondensiert, oder

b) ein reaktionsfähiges funktionelles Derivat einer Carbonsäure der Fomel V,

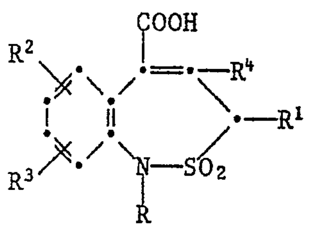

(V)

worin R, $R^1$, $R^2$, $R^3$ und $R^4$ die vorstehend angegebenen Bedeutungen aufweisen, mit einem Amin der Formel VI,

$Ar\text{-}(CH_2)_n\text{-}NH_2$ (VI)

worin Ar und n die vorstehend angegebenen Bedeutungen aufweisen, kondensiert, oder

c) für Verbindungen der Formel I, worin $R^4$ Hydroxy bedeutet, ein reaktionsfähiges funktionelles Derivat einer Carbonsäure der Formel VII,

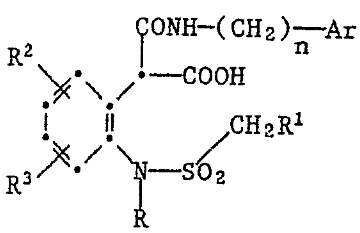

(VII)

worin R, $R^1$, $R^2$, $R^3$, n und Ar die vorstehend angegebenen Bedeutungen aufweisen, cyclisiert, oder

d) für Verbindungen der Formel I, worin $R^4$ Hydroxy bedeutet, ein reaktionsfähiges funktionelles Derivat einer Carbonsäure der Formel VIII,

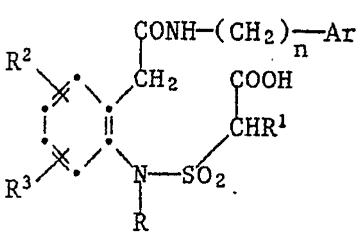

(VIII)

worin R, $R^1$, $R^2$, $R^3$, n und Ar die vorstehend angegebenen Bedeutungen aufweisen, cyclisiert, oder

e) für Verbindungen der Formel I, worin $R^4$ Hydroxy bedeutet, ein Enamin der Formel IX,

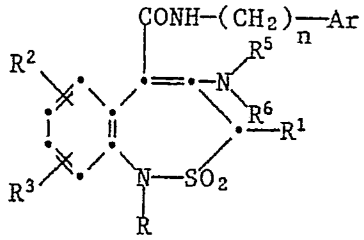

(IX)

worin R, $R^1$, $R^2$, $R^3$, $R^5$, $R^6$, n und Ar die vorstehend angegebenen Bedeutungen aufweisen, hydrolysiert, und in irgendeinem der vorstehenden Verfahren, wenn notwendig, eine erhaltene Verbindung der Formel I in eine andere Verbindung der Formel I überführt, und gegebenenfalls eine erhaltene Verbindung in ein Salz oder ein Salz in ein anderes Salz überführt oder die freie Verbindung aus einem erhaltenen Salz freisetzt.

In den Ausgangsmaterialien und Zwischenprodukten, die in die erfindungsgemässen Verbindungen übergeführt werden, wie es hierin beschrieben ist, sind funktionelle Gruppen, wie Amino oder Hydroxy, gewünschtenfalls durch konventionelle Schutzgruppen geschützt, die üblicherweise in der präparativen organischen Chemie verwendet werden. Geschützte Amino- und Hydroxygruppen sind solche, die unter milden Bedingungen in freie Amino- und Hydroxygruppen übergeführt werden können, ohne dass das molekulare Gerüst zerstört wird oder dass andere unerwünschte Nebenreaktionen ablaufen.

Bekannte Schutzgruppen, die diese Bedingungen erfüllen, sowie ihre Einführung und Abspaltung werden beispielsweise in J.F.W. McOmie, "Protective Groups in Organic Chemistry", Plenum Press, London, New York 1973, T.W. Green, "Protective Groups in Organic Synthesis", Wiley, New York 1984 beschrieben.

In den hierin angesprochenen Verfahren bedeuten reaktionsfähige funktionelle Derivate von Carbonsäuren beispielsweise Anhydride, insbesondere gemischte Anhydride, Säurehalogenide, Säureazide, Niederalkylester und aktivierte Ester davon. Gemischte Anhydride sind vorzugsweise solche von Pivalinsäure, oder ein Niederalkyl-(wie Ethyl oder Isobutyl) hemiester von Kohlensäure. Säurehalogenide sind beispielsweise Chloride oder Bromide. Aktivierte Ester sind beispielsweise Succinimido-, Phthalimido- oder 4-Nitrophenylester. Niederalkylester sind beispielsweise Methyl- oder Ethylester.

Die Kondensation einer freien Carbonsäure mit einem Amin der Formel VI in irgendeinem der hierin beschriebenen Verfahren kann in Gegenwart eines Kondensationsmittels ausgeführt werden, z.B. von Diethylphosphorcyanidat, 1,1′-Carbonyldiimidazol oder Dicyclohexylcarbodiimid, in einem inerten polaren Lösungsmittel, wie Dimethylformamid oder Dichlormethan.

Ein reaktionsfähiges verestertes Derivat eines Alkohols in irgendeinem der hierin angesprochenen Verfahren bedeutet besagten Alkohol verestert mit einer starken Säure, insbesondere einer starken anorganischen Säure, wie einer Halogenwasserstoffsäure, insbesondere Chlorwasserstoff, Bromwasserstoff oder Iodwasserstoffsäure, oder Schwefelsäure, oder einer starken organischen Säure, insbesondere einer starken organischen Sulfonsäure, wie einer aliphatischen oder aromatischen Sulfonsäure, z.B. Methansulfonsäure, 4-Methylphenylsulfonsäure oder 4-Bromphenylsulfonsäure. Ein derartiges reaktionsfähiges verestertes Derivat ist beispielsweise Halogen, z.B. Chlor, Brom oder Iod, oder aliphatisch oder aromatisch substituiertes Sulfonyloxy z.B. Methylsulfonyloxy oder 4-Methylphenylsulfonyloxy (Tosyloxy).

Die Kondensation eines Isocyanats der Formel IV mit einem Keton der Formel III gemäss Verfahren a) kann beispielsweise in Gegenwart einer anorganischen oder organischen Base durchgeführt werden, wie Natriumhydrid oder Triethylamin in einem polaren Lösungsmittel, wie einem Ether, z.B. Diethylether oder Tetrahydrofuran, und/oder einem Amid oder Sulfoxid, z.B. Dimethylformamid oder Dimethylsulfoxid, vorzugsweise bei einer Temperatur zwischen 25 und 100°C.

Die Kondensation eines Isocyanats der Formel IV mit einem Enamin der Formel IIIa gemäss Verfahren a) kann in einem inerten Lösungsmittel wie Toluol ausgeführt werden, vorzugsweise bei oder in der Nähe von Raumtemperatur.

In einer bevorzugten Ausführungsform von Verfahren a) zur Herstellung von Verbindungen der Formel I, worin $R^4$ Hydroxy bedeutet, wird zuerst ein Keton der Formel III in das entsprechende Enamin der Formel IIIa übergeführt, wobei Verfahren verwendet werden, die in der Technik bekannt sind, z.B. durch Behandlung mit einem sekundären Amin, vorzugsweise mit einem cyclischen Niederalkylenimin, insbesondere Pyrrolidin, in einem inerten mit Wasser nicht mischbaren Lösungsmittel, wie Toluol, in einer inerten Atmosphäre, gegebenenfalls unter Vewendung eines sauren Katalysators, wie p-Toluolsulfonsäure, vorzugsweise beim Siedepunkt der Lösungsmittels, so dass das freigesetzte Wasser vom Reaktionsgemisch abgetrennt werden kann. Das erhaltene Enamin wird dann, vorteilhafterweise in situ, mit einem Isocyanat der Formel IV, vorteilhafterweise bei Raumtemperatur, kondensiert, und ergibt ein Enaminderivat einer erfindungsgemässen Verbindung, z.B. einer Verbindung der Formel IXa,

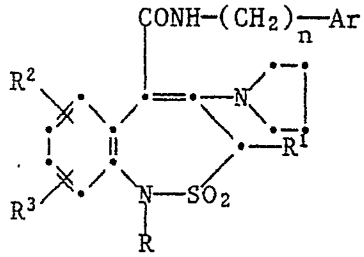

(IXa)

worin R, $R^1$, $R^2$, $R^3$, n und Ar die vorstehend angegebenen Bedeutungen aufweisen, welche wiederum gemäss Verfahren e) zu einer Verbindung der Formel I hydrolysiert wird, worin $R^4$ Hydroxy bedeutet, durch Behandlung mit z.B. wässriger Säure, wie mit Salzsäure.

Die Ausgangsverbindungen der Formel III werden vorteilhafterweise wie folgt hergestellt: Ein Niederalkylester einer in geeigneter Weise ortho-($R^1$-$CH_2SO_2NH$)-substituierten Phenylessigsäure wird mit einem reaktionsfähigen veresterten Derivat eines Alkohols, der dem Rest R entspricht (wenn R nicht Wasserstoff bedeutet), z.B. mit einem Halogenderivat davon, unter Standard-Alkylierungsbedingungen, z.B. in Dimethylformamid oder Tetrahydrofuran, in Gegenwart einer Base wie Kaliumcarbonat, umgesetzt. Man erhält so einen Niederalkylester einer Carbonsäure der Formel X,

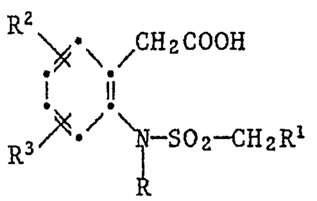

(X)

worin R und $R^1$-$R^3$ die vorstehend angegebenen Bedeutungen aufweisen.

Dieser Niederalkylester (oder ein anderes reaktionsfähiges funktionelles Derivat) der Carbonsäure der Formel X wird dann in Gegenwart einer starken Base cyclisiert, wie von Natriumhydrid in einem wasserfreien inerten polaren Lösungsmittel, wie Tetrahydrofuran, so dass das entsprechend substituierte bicyclische Keton der Formel III erhalten wird.

Die Kondensation gemäss Verfahren b) kann durchgeführt werden, indem vorteilhafterweise ein Niederalkylester als reaktionsfähiges funktionelles Derivat der Carbonsäure der Formel V verwendet wird. Die Amidbildung wird gemäss Standardverfahren, die im Stand der Technik bekannt sind, durchgeführt, indem z.B. der Ester mit einem Amin der Formel VI bei Rückflusstemperatur in einem Lösungsmittel wie Toluol oder Xylol kondensiert wird, vorteilhafterweise unter azeotroper Entfernung des Niederalkanols, der freigesetzt wird.

Ausgehend von einem Ester einer Verbindung der Formel V und abhängig von den Bedingungen und der Menge des Amins der Formel VI, welches verwendet wird, erhält man entweder eine Verbindung de Formel I, worin $R^4$ Hydroxy bedeutet, oder eine Verbindung der Formel I, worin $R^4$ $NH(CH_2)_n$–Ar bedeutet, worin n und Ar die vorstehend angegebenen Bedeutungen haben, oder ein Gemisch dieser Verbindungen.

Ein Ausgangsmaterial, z.B. ein Niederalkylester, a.B. der Ethylester einer Carbonsäure der Formel V wird vorzugsweise hergestellt, indem zuerst beispielsweise ortho-Aminophenylessigsäureethylester unter Standardbedingungen mit beispielsweise einer Verbindung der Formel XI

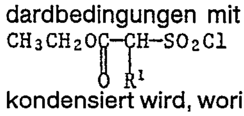

(XI)

kondensiert wird, worin $R^1$ die vorstehend angegebenen Bedeutungen hat. Man erhält so eine Verbindung der Formel XII,

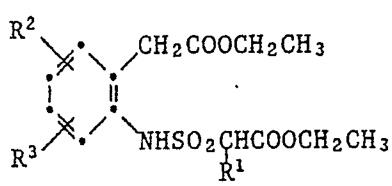

(XII)

worin $R^1$, $R^2$ und $R^3$ die vorstehend angegebenen Bedeutungen aufweisen, die dann zuerst mit einem reaktionsfähigen veresterten Derivat eines Alkohols, der dem Rest R entspricht (wenn R nicht Wasserstoff bedeutet) behandelt wird, in Gegenwart von beispielsweise Kaliumcarbonat, und danach mit einer starken Base, wie Natriumethanolat, in einem wasserfreien Medium cyclisiert wird. Man erhält so den Ethylester der entsprechenden Verbindung der Formel V, worin $R^4$ Hydroxy bedeutet.

Die entsprechenden Ausgangsverbindungen, worin $R^4$ für $NR^5R^6$ steht, können durch Kondensation mit dem entsprechenden Amin erhalten werden unter Bedingungen einer Enaminbildung und Abtrennung der

derivatisierten Verbindung von einer entstehenden Mischung.

Die Cyclisierung gemäss Verfahren c) kann vorteilhafterweise ausgeführt werden, indem ein Niederalkylester der Carbonsäure der Formel VII mit einer starken Base behandelt wird, z.B. mit Natriumhydrid in einem wasserfreien polaren Lösungsmittel wie Dimethylformamid, wie es für Zwischenprodukte unter Verfahren a) beschrieben ist.

Die Ausgangsverbindungen können hergestellt werden, wie nachstehend illustriert. Ortho-Nitrophenylmalonsäurediethylester wird zuerst mit einem Moläquivalent eines Amins der Formel VI kondensiert, gemäss der allgemeinen Verfahrensweise des Verfahrens b), das erhaltene Hemiester-Hemiamid wird dann zum entsprechenden Anilin hydriert, welches dann zuerst mit einem Aldehyd behandelt werden kann, der dem Rest R entspricht (wenn R nicht Wasserstoff bedeutet), in der Anwesenheit von beispielsweise Natriumcyanborhydrid als Reduktionsmittel, und dann mit einer Verbindung der Formel $R^1$-$CH_2SO_2Cl$ unter Standardbedingungen umgesetzt wird, so dass man den Ethylester einer Verbindung der Formel VII erhält.

Di Cyclisierung gemäss Verfahren d) kann ausgeführt werden, indem ein reaktionsfähiger Ester, z.B. der Ethylester einer Verbindung der Formel VIII, mit einer starken Base behandelt wird, z.B. mit zwei Aequivalenten Butyllithium.

Die Ausgangsmaterialien, z.B. der Ethylester einer Verbindung der Formel VIII, können wie folgt hergestellt werden: Das entsprechend N-substituierte ortho-Nitrophenylacetamid wird zuerst zum entsprechenden Anilin reduziert, welches dann zuert in das R-substituierte sekundäre Amin umgewandelt werden kann, beispielsweise durch Behandlung mit dem dem Rest R entsprechenden Aldehyd (wenn R nicht Wasserstoff bedeutet), in Gegenwart von beispielsweise Natriumcyanborhydrid, und dann kann besagtes Amin mit einer Verbindung der Formel IX kondensiert werden.

Die Hydrolyse gemäss Verfahren e) wird vorzugsweise durch Behandlung mit einer wässrigen Säure, wie mit Salzsäure, durchgeführt. Die Ausgangsverbindungen der Formel IX werden z.B. hergestellt, wie es für Verbindungen der Formel IXa bei Verfahren a) beschrieben ist, oder wie bei Verfahren b) und in den Beispielen beschrieben.

Weitere Ausgangsmaterialien, beispielsweise der Formeln IV und VI, sind entweder bekannt oder werden gemäss an sich bekannten Verfahren hergestellt.

Die erfindungsgemässen Verbindungen, die sich so erhalten lassen, können auf an sich bekannte Weisen ineinander übergeführt werden.

So können beispielsweise erhaltene Verbindungen der Formel I, worin $R^4$ Hydroxy bedeutet (als Enole), mit beispielsweise Niederdiazoalkanen verethert werden, oder verestert, z.B. mit Niederalkancarbonsäureanhydriden.

Verbindungen der Formel I, worin R Wasserstoff bedeutet, insbesondere solche Verbindungen, worin $R^4$ Hydroxy bedeutet, können in verbindungen der Formel I übergeführt werden, worin R ein Substituent wie vorstehend definiert ist, unter Bedingungen, die im Stand der Technik für die Substitution eines Sulfonamides bekannt sind, z.B. durch Behandlung mit einem reaktionsfähigen veresterten Derivat eines Alkohols, der dem Rest R entspricht, z.B. einem Halogenid, unter basischen Bedingungen, beispielsweise in Anwesenheit einer Base wie Kalium- oder Natriumcarbonat, in einem polaren Lösungsmittel wie Dimethylformamid.

Verbindungen der Formel I, worin $R^4$ für $NR^5R^6$ steht, können in Verbindungen der Formel I übergeführt werden, worin $R^4$ Hydroxy bedeutet, wie dies unter Verfahren e) beschrieben ist.

Die vorstehend beschriebenen Umsetzungen werden im übrigen gemäss Standardverfahren ausgeführt, in Anwesenheit oder Abwesenheit von Verdünnungsmitteln, vorzugsweise solchen, die gegenüber den Reagenzien inert sind und diese zu lösen vormögen, von Katalysatoren, Kondensations- oder Neutralisationsmitteln und/oder inerten Atmosphären, bei niedrigen Temperaturen, Raumtemperatur oder erhöhten Temperaturen, bei atmosphärischem oder superatmosphärischem Druck.

Die Erfindung betrifft ebenfalls Abänderungen der vorliegenden Verfahren, wonach ein auf irgendeiner Stufe des Verfahrens-erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet oder worin ein Ausgangsstoff in Form eines Salzes oder eines reaktionsfähigen Derivates verwendet wird. In den genannten Reaktionen werden vorteilhafterweise solche Ausgangsstoffe verwendet, welche zu den weiter vorn als bevorzugt beschriebenen Verbindungen führen.

Die Erfindung betrifft auch neue Zwischenprodukte und Verfahren zu ihrer Herstellung.

Saure erfindungsgemässe Verbindungen können mit pharmazeutisch annehmbaren Basen in Salze übergeführt werden, z.B. mit wässrigen Alkalimetallhydroxiden, vorzugsweise in Anwesenheit eines etherischen oder alkoholischen Lösungsmittels, wie eines Niederalkanols. Aus den Lösungen letzterer können die Salze mit einem Ether ausgefällt werden, z.B. mit Diethylether. Basische erfindungsgemässe Verbindungen können in Säureadditionssalze übergeführt werden, z.B. durch Behandlung mit einer geeigneten Säure, z.B. in alkoholischer Lösung. Entsprechende Salze können in die freien Verbindungen durch Behandeln mit Säuren oder Basen übergeführt werden. Diese oder andere Salze können auch zur Reinigung der erhaltenen Verbindungen verwendet werden.

In Anbetracht der engen Beziehungen zwischen den freien Verbindungen und den Verbindungen in Form ihrer Salze sind in diesem Zusammenhang unter freien Verbindungen sinn- und zweckgemäss gegebenenfalls immer auch die entsprechenden Salze zu verstehen.

Die Verbindungen der Erfindung und ihre Salze können auch in Form ihrer Hydrate erhalten werden, oder sie

können andere zur Kristallisation verwendete Lösungsmittel einschliessen.

Falls Gemische einer der vorstehend genannten Verbindunge oder von Zwischenprodukten erhalten werden, können diese durch an sich bekannte Methoden in die einzelnen Verbindungen aufgetrennt werden, z.B. durch fraktionierte Destillation, Kristallisation oder Chromatographie.

Racemische Produkte der Erfindung oder entsprechende Zwischenprodukte können in die optischen Antipoden aufgetrennt werden, beispielsweise durch Trennung von diastereomeren Salzen, die mit optisch aktiven Säuren oder Basen gebildet werden.

Die vorliegende Erfindung bezieht sich auch auf pharmazeutische Präparate, insbesondere pharmazeutische Präparate, die für die Behandlung und Heilung von Entzündungskrankheiten geeignet sind, z.B. von rheumatischer Arthritis und Osteoarthritis in Säugern.

Die erfindungsgemässen pharmazeutischen Präparate sind solche, die für enterale, wie orale oder rektale, transdermale und parenterale Verabreichung an Säuger, unter Einschluss des Menschen, zur Erleichterung und Behandlung von inflammatorischen und arthritischen Krankheiten, wie Osteoarthritis und rheumatischer Arthritis geeignet sind, und eine wirksame Menge einer pharmakologisch aktiven erfindungsgemässen Verbindung in Kombination mit einem oder mehreren pharmazeutisch annehmbaren Träger enthalten.

Die pharmakologisch aktiven Verbindungen der Erfindung sind bei der Herstellung von pharmazeutischen Präparaten verwendbar, die eine wirksame Menge derselben in Verbindung oder Beimischung mit Excipienten oder Trägern, die für die enterale oder parenterale Anwendung geeignet sind, umfassen. Bevorzugt sind Tabletten und Gelatinekapseln, die den aktiven Bestandteil zusammen mit a) Verdünnungsmitteln, z.B. Lactose, Dextrose, Sucrose, Mannit, Sorbit, Cellulose und/oder Glycin, b) Gleitmitteln, z.B. Siliciumdioxid, Talk, Stearinsäure, deren Magnesium- oder Calciumsalze und/oder Polyethylenglycol, für Tabletten auch c) Bindemitteln, z.B. Magnesiumaluminiumsilicat, Stärkepaste, Gelatine, Tragant, Methylcellulose, Natriumcarboxymethylcellulose und/oder Polyvinylpyrrolidon, gewünschtenfalls d) Zerteilungs- bzw. Desintegrationsmitteln, z.B. Stärken, Agar, Alginsäure oder deren Natriumsalz, oder schäumenden Mischungen und/oder e) Absorbentien, farbgebenden Mitteln, Geschmacksstoffen und süssenden Mitteln umfassen. Injizierbare Präparate sind vorzugsweise wässrige isotonische Lösungen oder Suspensionen, und Suppositorien werden vorteilhaft aus Fettemulsionen oder -suspensionen hergestellt. Diese Zusammensetzung können sterilisiert werden und/oder Adjuvanzien, wie Konservierungs-, Stabilisierungs-, Netz- oder Emulgiermittel, Lösungspromotoren, Salze für die Regulierung des osmotischen Drucks und/oder Puffer enthalten. Zusätzlich können sie auch andere therapeutische wertvolle Substanzen enthalten. Diese Präparate werden nach herkömmlichen Misch-, Granulier- bzw. Ueberzugsmethoden hergestellt und enthalten etwa 0,1 bis 75 %, vorzugsweise etwa 1 bis 50 % des aktiven Bestandteils.

Geeignete Formulierungen für die transdermale Anwendung enthalten eine wirksame Menge einer erfindungsgemässen Verbindung zusammen mit Trägermaterial. Geeignete Trägermaterialien umfassen pharmazeutisch annehmbare Hilfsstofe, welche den Durchgang durch die Haut ermöglichen. Insbesondere haben transdermale therapeutische Systeme die Form eines Pflasters mit einer Schutzschicht, einem Wirkstoffreservoir, gegebenenfalls mit Trägermaterialien, und einer die Abgabe bestimmenden Kontrollschicht, durch welche der Wirkstoff auf die Haut mit kontrollierter und bestimmbarer Geschwindigkeit über einen längeren Zeitabschnitt abgegeben wird. Das System hat gegebenenfalls noch eine Klebschicht.

Die vorliegende Erfindung bezieht sich weiterhin auf ein Verfahren zur Inhibierung der chemotaktischen Aktivierung von neutrophilen Granulozyten, oder zur Inhibierung des Abbaus von Knorpelgrundsubstanz in Säugern, und zur Behandlung von Krankheiten, welche darauf ansprechen, welches darin besteht, dass einem Säuger, der dessen bedarf, eine wirksame Menge einer erfindungsgemässen Verbindung oder eines pharmazeutischen Präparates, das eine solche Verbindung in Kombination mit einem oder mehreren pharmazeutisch annehmbaren Trägern enthält, verabreicht wird. Die Erfindung bezieht sich ferner insbesondere auf ein Verfahren zur Behandlung und Heilung von inflammatorischen und arthritischen Krankheiten in Säugern, wie rheumatischer Arthritis, welches darin beteht, dass einem Säuger, der dessen bedarf, eine wirksame menge einer Verbindung der Erfindung oder eines pharmazeutischen Präparates, das eine erfindungsgemässe Verbindung in Kombination mit einem oder mehreren pharmazeutisch annehmbaren Trägern enthält, verabreicht wird.

Die Dosis der Wirksubstanzen, die verabreicht wird, hängt ab von der Spezies des Warmblüters (Säugers), dem Körpergewicht, Alter und individuellen Zustand sowie der Art der Verabreichung.

Ein Einheitsdosis für einen Säuger von etwa 50 bis 70 kg kann zwischen etwa 5 und 100 mg der Wirksubstanz enthalten.

Die folgenden Beispiele sollen die Erfindung näher erläutern und sind nicht geeignet, sie in irgendeiner Weise einzuschränken. Temperaturen werden in Grad Celsius angegeben. Wenn nicht anders angegeben, werden alle Verdapfungen unter vermindertem Druck ausgeführt, vorzugsweise zwischen etwa 2 und 13 kPa. Die Struktur von Endprodukten, Zwischenprodukten und Ausgangsverbindungen ist z.B durch analytische Verfahren und spektroskopische Charakteristika gesichert (z.B. MS, IR, NMR).

Bei der Benennung der erfindungsgemässen Verbindungen, d.h. derer der Formel I, bezieht sich der N-(Stickstoff)-Substituent auf die Gruppe $(CH_2)_n$-Ar, die an das Stickstoffatom der $CONH(CH_2)_n$-Ar-Gruppe in Formel I gebunden ist.

Beispiel 1:

Ein Gemisch von 1-(4-Chlorphenylmethyl)-1,5-dihydro-2,1-benzothiazepin-4(3H)-on-2,2-dioxid (1.97 kg) und Pyrrolidin (421 g) in Toluol (9,9 l) wird 3 Stunden unter einer Stickstoffatmosphäre gerührt und unter Rückfluss erhitzt, und das gebildete Wasser wird in einer Dean-Stark-Falle gesammelt. Die Lösung wird in einem Eisbad auf 5° C gekühlt. 4-Chlorphenylisocyanat (995 g) wird auf einmal unter Rühren zugegeben, welches bei Raumtemperatur 16 Stunden fortgesetzt wird. Das Gemisch wird in einem Eisbad auf 5° C gekühlt, filtriert und mit Toluol (1,0 l) gewaschen. Der Filterkuchen wird über Nacht luftgetrocknet. Man erhält so das N-(4-Chlorphenyl)-1-(4-chlorphenylmethyl)-1,3-dihydro-4-(1-pyrrolidinyl)-2,1-benzothiazepin-5-carboxamid-2,2-dioxid, Fp. 190-194° C, die Verbindung der Formel I, worin $R^1$, $R^2$ und $R^3$ Wasserstoff bedeuten, R für 4-Chlorphenylmethyl steht, n für null steht, Ar 4-Chlorphenyl bedeutet und $R^4$ 1-Pyrrolidinyl bedeutet.

Das Ausgangsmaterial wird wie folgt hergestellt: Zu einer Lösung von 2-Nitrophenylessigsäure (2,00 kg) in Methanol (4 l) wird bei Raumtemperatur konzentrierte Schwefelsäure (71,0 ml) auf einmal zugegeben. Die Lösung wird gerührt und unter Rückfluss erhitzt für 6 Stunden, dann lässt man über Nacht bei Raumtemperatur stehen. Nach Kühlen in einem Eisbad auf 7° C wird die Lösung durch Zugabe von konzentriertem Ammoniumhydroxid (150 ml) auf pH 8 eingestellt, wobei die Temperatur unter 10° C gehalten wird. Die Lösung wird im Vakuum auf ein Volumen von 2 l eingeengt und dann mit Dichlormethan (2,5 l) gemischt. Die vereinigte Lösung wird mit Wasser (2 x 500 ml) gewaschen sowie mit gesättigter Kochsalzlösung (500 ml), über wasserfreiem Magnesiumsulfat getrocknet und zur Trockne eingeengt. Man erhält ein Rohprodukt, welches destilliert wird und so den 2-Nitrophenylessigsäuremethylester, Kp. 116-131° C (26 Pa) gibt.

Zu einer Lösung von 2-Nitrophenylessigsäuremethylester (6,04 kg) in Toluol (100 l) wird wasserfreies Magnesiumsulfat (2,00 kg) gegeben, danach 5 % Palladium auf Kohle (125 g). Das Gemisch wird gerührt und hydriert, wobei die Temperatur unter 40° C gehalten wird, indem die Rührgeschwindigkeit und die Zudosierung von Wasserstoffgas reguliert werden. Nach beendeter Zugabe wird das Gemisch filtriert. Das Filtrat, welches 2-Aminophenylessigsäuremethylester enthält, wird auf -20° C abgekühlt, Triethylamin (3,75 kg) wird unter Rühren auf einmal zugegeben. Danach wird langsam eine Lösung von Methansulfonylchlorid (3,91 kg) in Toluol (4,5 l) zugegeben, wobei die Temperatur zwischen -2° und 5° C gehalten wird. Nach 1 Stunde Rühren bei -2° C wird das Gemisch filtriert und mit Toluol (10 l) gewaschen. Der Filterkuchen wird dann 90 Minuten mit Wasser versetzt (90 l), filtriert und mit Wasser (45 l) und Methanol (8 l) gewaschen. Der Feststoff wird bei 50° C über Nacht im Vakuum getrochnet. Man erhält so den 2-(Methylsulfonylamino)phenylessigsäuremethylester, Fp. 73-75° C.

Zu einer Lösung von 2-(Methylsulfonylamino)phenylessigsäuremethylester (2,00 kg) in Dimethylformamid (10 l) wird unter einer Stickstoffatmosphäre 4-Chlorbenzylchlorid (1,37 kg) auf einmal gegeben, gefolgt von wasserfreiem Kaliumcarbonat (1,18 kg). Das Gemisch wird auf 75° C erhitzt und 4 Stunden gerührt, dann über Nacht auf Raumtemperatur abgekühlt, worauf es in Wasser (50 l) gegeben wird. Der erhaltene Niederschlag wird in Dichlormethan (4 l) aufgelöst. Die Dichlormethanlösung wird mit gesättigter Kochsalzlösung (500 ml) gewaschen, über wasserfreiem Magnesiumsulfat getrocknet und filtriert. Das Filtrat wird im Vakuum eingeengt, man erhält einen Feststoff, der über Nacht luftgetrocknet und dann bei Raumtemperatur vakuumgetrocknet (40 Pa) wird. Man erhält so den 2-[N-(4-Chlorphenylmethyl)-methylsulfonylamino]-phenylessigsäuremethylester, Fp. 73-75° C.

Zu einer Lösung von 2-[N-(4-Chlorphenylmethyl)-methylsulfonylamino]-phenylessigsäuremethylester (1,00 kg) in trockenem Tetrahydrofuran (8 l) wird Natriumhydrid (60 % in Oel, 120 g) unter einer Stickstoffatmosphäre auf einmal zugegeben. Das Gemisch wird gerührt und unter Rückfluss erhitzt während etwa 50 Minuten. Nach weiteren 40 Minuten Erhitzens unter Rückfluss beginnt eine Wasserstoffgasentwicklung, welche ungefähr 5 - 10 Minuten andauert. Zu diesem Zeitpunkt ergibt sich eine vollständige Lösung, und es wird weitere 15 Minuten unter Rückfluss erhitzt. Die Lösung wird in einem Eisbad auf 5° C abgekühlt, und Eisessig (172 ml) wird während 15 Minuten dazugegeben, wobei die Temperatur unter 10° C gehalten wird, um pH 6 einzustellen. Das Lösungsmittel wird im Vakuum bei 40 - 50° C abgezogen, und der Rückstand wird zwischen Dichlormethan (4 l) und Waser (1 l) verteilt. Die Phasen werden getrennt, und die organische Phase wird mit Wasser (1 l) nd gesättigter Kochsalzlösung (1 l) gewaschen. Nach Trocknen über wasserfreiem Magnesiumsulfat wird die Lösung filtriert, und das Filtrat wird im Vakuum eingeengt auf ein Volumen von 1,2 l und im Eisbad gekühlt. Der Feststoff wird abfiltriert, und der Filterkuchen wird mit wassserfreiem Ether (300 ml) gewaschen. Man erhält so, nach Trocknen an der Luft über Nacht, 1-(4-Chlorphenylmethyl)-1,5-dihydro-2,1-benzothiazepin-4(3H)-on-2,2-dioxid, Fp. 147-148° C.

Beispiel 2:

a) Eine Lösung von 1,50 kg N-(4-Chlorphenyl)-1-(4-chlorphenylmethyl)-1,3-dihydro-4-(1-pyrrolidinyl)-2,1-benzothiazepin-5-carboxamid-2,2-dioxid in Dichlormethan (15,0 l) wird mit 6 N Salzesäure (1,43 l) vermischt, und das zweiphasige Gemisch wird gerührt und über Nacht unter Rückfluss erhitzt. Die Phasen ewrden getrennt, und die organische Phase wird mit Wasser (4,0 l) und gesättigter Kochsalzlösung (2,0 l) gewaschen, über wasserfreiem Magnesiumsulfat getrocknet und filtriert. Das Filtrat wird im Vakuum auf ein Volumen von 3,5 l eingeengt, und zu diesem wird eine gleiche Menge Ether zugegeben. Das Gemisch wird 15 Minuten gerührt, filtriert, und der Filterkuchen wird mit wasserfreiem Ether (500 ml) gewaschen. Das Produkt wird über Nacht bei 105-110° C/40 Pa getrocknet. Man erhält so N-(4-Chlorphenyl)-1-(4-chlorphenylmethyl)-1,3-dihydro-4-hydroxy-2,1-benzothiazepin-5-carboxamid-2,2-dioxid, Fp. 182-183°C als Hydrat, welches 1/2 Moläquivalent Wasser enthält, und welches die Verbindung der Formel II ist, worin $R^1$, $R^2$ und $R^3$ Wasserstoff bedeuten, R für 4-Chlorphenylmethyl steht und Ar 4-Chlorphenyl bedeutet.

b) Eine Aufschlemmung von 814,0 g N-(4-Chlorphenyl)-1-(4-chlorphenylmethyl)-1,3-dihydro-4-hydroxy-2,1-benzothiazepin-5-carboxamid-2,2-dioxid in absolutem Ethanol (3,0 l) wird unter einer Stickstoffatmosphäre bei Raumtemperatur gerührt, während eine Lösung von Natriumhydroxid (65,4 g) in Wasser (163 ml) in einem dünnen Strom während eines Zeitraums von 20 Minuten zugegeben wird. Es wird weiterhin 1,5 Stunden gerührt, und die kleine Menge unlöslichen Materials wird durch Filtration durch ein Filter-Cel-Filter entfernt. Das Filtrat wird bei 40 - 50°C unter vermindertem Druck zur Trockene eingeengt, und der Rückstand wird bei Raumtemperatur (300-400 Pa) über Nacht weiter getrocknet. Der weisse Schaum, der erhalten wird, wird in einer Mühle gemahlen, wobei ein 1,0 mm Sieb benutzt wird. Das fein gepulverte Produkt wird bei Raumtemperatur 6 Tage getrocknet. Man erhält so N-(4-Chlorphenyl)-1-(4-chlorphenylmethyl)-1,3-dihydro-4-hydroxy-2,1-benzothiazepin-5-carboxamid-2,2-dioxid-Natriumsalz, Fp. 209-210° C (Zersetzung) als Hydrat, welches 1,5 Moläquivalente Wasser enthält.

Auf ähnliche Weise werden das Zinksalz, Fp. >250°C, das Kupfersalz, Fp. >250°C und das Magnesiumsalz, Fp. 394-395°C (Zersetzung) hergestellt.

Beispiel 3:

Eine gerührte Mischung von 3 g 1-(4-Chlorphenylmethyl)-1,3-dihydro-4-hydroxy-2,1-benzothiazepin-5-carbonsäure-2,2-dioxidethylester, 1,04 g 4-Chloranilin und 100 ml Toluol wird 18 Stunden unter Rückfluss erhitzt. Nach Kühlen auf Raumtemperatur wird das Lösungsmittel unter vermindertem Druck abgezogen, und der Rückstand wird in 200 ml Ether gelöst, mit Aktivkohle behandelt, filtriert und Mit Hexan verdünnt. Daraufhin kristallisiert N-(4-Chlorphenyl)-1-(4-chlorphenylmethyl)-1,3-dihydro-4-hydroxy-2,1-benzothiazepin-5-carboxamid-2,2-dioxid aus und wird durch Filtration gesammelt. Es weist einen Schmelzpunkt von 182-183°C auf.

Das Ausgangsmaterial wird wie folgt hergestellt: Zu einer Lösung von 82,7 g 2-Aminophenylessigsäuremethylester (siehe Beispiel 1) in 1,2 l Toluol werden 42 ml Pyridin gegeben, und das Gemisch wird bei Raumtemperatur gerührt. Zu diesem Gemisch wird eine Lösung von 93,2 g Ethoxycarbonylmethylsulfonylchlorid in 150 ml Toluol tropfenweise während 30 Minuten gegeben, wobei die Temperatur des Ansatzes mit Hilfe eines Eis-Wasserbades unterhalb von 10°C gehalten wird. Nach Rühren bei 0°C für eine weitere Stunde wird das Gemisch in 50 ml Eiswasser gegeben. Die organische Phase wird abgetrennt und mit 150 ml kalter verdünnter Salzsäure gewaschen, danach mit 150 ml gesättiger Natriumchloridlösung. Der Toluolextrakt wird über wasserfreiem Magnesiumsulfat getrocknet, filtriert, und das Lösungsmittel unter vermindertem Druck abgezogen. Man erhält so den 2-(Ethoxycarbonylmethylsulfonylamino)phenylessigsäuremethylester als Oel, der im nächsten Schritt ohne weitere Reinigung verwendet wird.

Zu einer Lösung von 54,6 g 2-(Ethoxycarbonylmethylsulfonylamino)phenylessigsäuremethylester in 300 ml Dimethylformamid unter einer Stickstoffatmosphäre werden 28 g 4-Chlorbenzylchlorid und danach 35 g wasserfreies Kaliumcarbonat zugegeben. Das Gemisch wird auf 75°C erhitzt und 1 Stunde gerührt, dann lässt man auf Raumtemperatur abkühlen, wonach das Gemisch in 1 l Wasser gegeben wird. Das Gemisch wird dann mit 2 x 300 ml Ether extrahiert, die vereinigten etherischen Extrakte werden mit 250 ml gesättigter Kochsalzlösung gewaschen, abgetrennt und über wasserfreiem Magnesiumsulfat getrocknet. Das Lösungsmittel wird unter vermindertem Druck abgezogen, und der Rückstand von 2-[N-(4-Chlorphenylmethyl)-ethoxycarbonylmethylsulfonylamino]-phenylessigsäuremethylester wird aus einem Gemisch von Ether-Hexan (4:1) kristallisiert; Fp. 86-88°C.

Eben dieses Zwischenprodukt kann auch auf folgende alternative Weise hergestellt werden:

a) Ein Gemisch von 31,5 g 2-Aminophenylessigsäuremethylester und 26,8 g 4-Chlorbenzaldehyd wird unter Hochvakuum auf 50°C erhitzt, um Wasser zu entfernen. 2-(4-Chlorphenylmethylenimino)phenylessigsäuremethylester wird als Oel erhalten, welches im nächsten Schritt ohne weitere Reinigung verwendet wird.

b) Zu einer gekühlten und gerührten Mischung von 52,5 g 2-(4-Chlorphenylmethylenimino)phenylessigsäuremethylester und 350 ml Essigester werden 180 ml kaltes Methanol, danach 9,2 g Natriumcyanborhydrid und 25 ml ethanolischer Chlorwasserstoffsäure gegeben. Es wird weitere 4 Stunden bei 0°C gerührt, und man lässt den Ansatz sich dann auf Raumtemperatur erwärmen. Das Lösungsmittel wird durch Abdampfen unter vermindertem Druck entfernt, und der Rückstand wird in 500 ml Eiswasser gegeben. Das Gemisch wird mit verdüntem Natriumhydroxid basisch gemacht und mit 3 x 350 ml Ether extrahiert. Die vereinigten etherischen Extrakte werden getrennt, mit 2 x 250 ml gesättigter Kochsalzlösung gewaschen, über wasserfreiem Magnesiumsulfat getrocknet, filtriert, und das Lösungsmittel wird unter vermindertem Druck abgezogen. Man enthält so 2-(4-Chlorphenylmethylamino)phenylessigsäuremethylester als Oel, welches im nächsten Schritt ohne weitere Reinigung verwendet wird.

c) Zu einer gerührten Lösung von 48 g 2-(4-Chlorphenylmethylamino)phenylessigsäuremethylester in 1,2 l Toluol, welche 26 ml Triethylamin enthält, werden 31 g Ethoxycarbonylmethylsulfonylchlorid in 50 ml Toluol tropfenweise während 15 Minuten zugegeben, wobei die Temperatur des Ansatzes unter 10°C gehalten wird. Man lässt das Gemisch dann über Nacht bei Raumtemperatur rühren und gibt es in 2 l Eiswasser und trennt die organische Schicht ab. Der Toluolextrakt wird mit 3 x 750 ml gesättiger Kochsalzlösung gewaschen, abgetrennt, über wasserfreuem Magnesiumsulfat getrocknet, filtriert, und das Lösungsmittel unter vermindertem Druck abgezogen. Man erhält so 40 g 2-[N-(4-Chlorphenylmethyl)-ethoxycarbonylmethylsulfonylamino]-phenylessigsäuremethylester, der aus Ether-Hexan (4:1) kristallisiert werden kann, Fp. 86-88° C.

Eine Lösung von Natriumethanolat wird durch Zugabe von 4 g Natriumstücken zu 550 ml wasserfreiem Ethanol hergestellt. Zu dieser Lösung werden 24 g 2-[N-(4-Chlorphenylmethyl)-ethoxycarbonylmethylsulfonylamino]-phenylessigsäuremethylester gegeben, und der Ansatz wird 18 Stunden unter Rückfluss erhitzt. Nach Abkühlen auf Raumtemperatur wird das Ethanol unter vermindertem Druck abgezogen, und der Rückstand wird in 750 ml Eiswasser gegeben, mit 5N Salzsäure angesäuert, und das Gemisch wird mit 3 x 500 ml Ether extrahiert. Die vereinigten etherischen Extrakte werden mit 2 x 350 ml gesättigter Kochsalzlösung gewaschen, abgetrennt, über wasserfreiem Magnesiumsulfat getrocknet, filtriert, und das Lösungsmittel wird unter vermindertem Druck abgezogen. Man erhält so 1-(4-Chlorphenylmethl)-1,3-dihydro-4-hydroxy-2,1-benzothiazepin-5-carbonsäure-2,2-dixidethylester, welcher aus Ethanol kristallisiert werden kann, Fp. 147-149° C.

Beispiel 4:

Folgende Verbindungen können auf analoge Weise zu den Verfahren, die in den vorherstehenden Beispielen beschrieben sind, hergestellt werden, wenn die entsprechenden Ausgangsmaterialien verwendet werden:

a) N-(2-Pyridyl)-1-(4-chlorphenylmethyl)-1,3-dihydro-4-hydroxy-2,1-benzothiazepin-5-carboxamid-2,2-dioxid, Fp. 235° C;

b) N-Phenyl-1-(4-chlorphenylmethyl)-1,3-dihydro-4-hydroxy-2,1-benzothiazepin-5-carboxamid-2,2-dioxid, Fp. 181-183° C;

c) N-(4-Methylthiophenyl)-1-(4-chlorphenylmethyl)-1,3-dihydro-4-hydroxy-2,1-benzothiazepin-5-carboxamid-2,2-dioxid, Fp. 183-185° C;

d) N-(3-Trifluormethylphenyl)-1-(4-chlorphenylmethyl)-1,3-dihydro-4-hydroxy-2,1-benzothiazepin-5-carboxamid-2,2-dioxid, Fp. 156-158° C;

e) N-(2-Thiazolyl)-1-(4-chlorphenylmethyl)-1,3-dihydro-4-hydroxy-2,1-benzothiazepin-5-carboxamid-2,2-dioxid, Fp. 175-177° C;

f) N-(4-Chlorphenyl)-1-(4-chlorphenylmethyl)-8-trifluormethyl-1,3-dihydro-4-hydroxy-2,1-benzothiazepin-5-carboxamid-2,2-dioxid, Fp. 74-75°;

g) N-(4-Chlorphenyl)-1-(4-chlorphenylmethyl)-7,8-dimethoxy-1,3-dihydro-4-hydroxy-2,1-benzothiazepin-5-carboxamid-2,2-dioxid, Fp. 248-250° C;

h) N-(4-Methylthiophenyl)-1-(4-chlorphenylmethyl)-8-trifluormethyl-1,3-dihydro-4-hydroxy-2,1-benzothiazepin-5-carboxamid-2,2-dioxid, Fp. 193-195° C;

i) N-(4-Chlorphenyl)-1-(4-chlorphenylmethyl)-3-methyl-1,3-dihydro-4-hydroxy-2,1-benzothiazepin-5-carboxamid-2,2-dioxid, Fp. 190-192° C;

j) N-(4-Chlorphenyl)-1-(4-chlorphenylmethyl)-3-(phenylmethyl)-1,3-dihydro-4-hydroxy-2,1-benzothiazepin-5-carboxamid-2,2-dioxid, Fp. 186-188°;

k) N-(4-Chlorphenyl)-1-(2-phenylethyl)-1,3-dihydro-4-hydroxy-2,1-benzothiazepin-5-carboxamid-2,2-dioxid, Fp. 179-181° C;

l) N-(4-Chlorphenyl)-1-(4-dichlorphenylmethyl)-1,3-dihydro-4-hydroxy-2,1-benzothiazepin-5-carboxamid-2,2-dioxid, Fp. 157-159° C;

m) N-(4-Chlorphenyl)-1-phenylmethyl-1,3-dihydro-4-hydroxy-2,1-benzothiazepin-5-carboxamid-2,2-dioxid, Fp. 182-184° C;

n) N-(4-Chlorphenyl)-1-(4-bromphenylmethyl)-1,3-dihydro-4-hydroxy-2,1-benzothiazepin-5-carboxamid-2,2-dioxid;

o) N-(2-Carboxy-4-chlorphenyl)-1-(4-chlorphenylmethyl)-1,3-dihydro-4-hydroxy-2,1-benzothiazepin-5-carboxamid-2,2-dioxid, Fp. 208-210° C;

p) N-(2-Thiazolyl)-1-methyl-1,3-dihydro-4-hydroxy-2,1-benzothiazepin-5-carboxamid-2,2-dioxid, Fp. 235-237° C;

q) N-(3,4-Dichlorphenylmethyl)-1-(4-chlorphenylmethyl)-1,3-dihydro-4-hydroxy-2,1-benzothiazepin-5-carboxamid-2,2-dioxid, Fp. 164-166° C;

r) N-(4-Chlorphenyl)-1-(4-chlorphenylmethyl)-3-(4-bromphenylmethyl)-1,3-dihydro-4-hydroxy-2,1-benzothiazepin-5-carboxamid-2,2-dioxid, Fp. 197-199° C.

Beispiel 5:

Ein Gemisch von 1,7 g 1-(3,4-Dichlorphenylmethyl)-1,3-dihydro-4-hydroxy-2,1-benzothiazepin-5-carbonsäure-2,2-dioxidethylester, 0,6 g 5-Chlorphenethylamin und 100 ml Toluol wird 2 Tage unter Rückfluss erhitzt. Der Ansatz wird zur Trockene eingeengt. Der Rückstand wird aus Ether-Hexan (Aktivkohle) kristalllisiert. Man erhält so ) N-(4-Chlorphenethyl)-1-(3,4-dichlorphenylmethyl)-1,3-dihydro-4-(4-chlorphenethylamino)-2,1-benzothiazepin-5-carboxamid-2,2-dioxid; Fp. 119-121° C.

Beispiel 6:

Herstellung von 1.000 Kapseln mit je 10 mg Wirkstoff:

| Bestandteile | |
|---|---|
| N-(4-Chlorphenyl)-1-(4-chlorphenylmethyl)-1,3-dihydro-4-hydroxy-2,1-benzothiazepin-5-carboxamid-2,2-dioxid | 10,00 g |
| Lactose | 207,00 g |
| Stärke | 80,00 g |
| Magnesiumstearat | 3,00 g |

Das pulverige Material wird durch ein Sieb mit Oeffnungen von 0,6 mm Durchmesser gedrückt. Anschliessend mischt man in einem Mischer den Wirkstoff mit dem Magnesiumstearat, dann mit der Lactose und der Stärke, bis man eine homogene Masse erhält. Man füllt Nr. 2 Hartgelatinekapseln mit jeweils 300 mg der vorbereiteten Mischung.

Analog kann man Kapseln, welche 1 - 50 mg einer der anderen vorstehend offenbarten Verbindungen enthalten, herstellen.

## Patentansprüche

1. Verbindungen der Formel I,

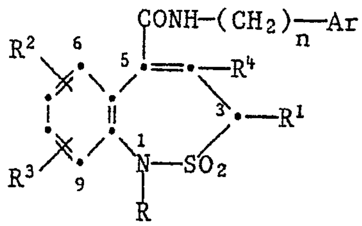

(I)

worin n null, eins oder zwei bedeutet; Ar einen carbocyclischen oder heterocyclischen Arylrest bedeutet; R und $R^1$ unabhängig voneinander Wasserstoff, Niederalkyl oder Ar-Niederalkyl bedeuten; $R^2$ und $R^3$ unabhängig voneinander Wasserstoff, Niederalkyl, Halogen, Trifluormethyl oder Niederalkoxy bedeuten; oder $R^2$ und $R^3$ an benachbarten Kohlenstoffatomen gemeinsam Niederalkylendioxy bedeuten; $R^4$ Hydroxy oder $NR^5R^6$ bedeutet, worin $R^5$ und $R^6$ unabhängig voneinander für Wasserstoff, Niederalkyl oder Ar-Niederalkyl stehen; oder worin $R^5$ und $R^6$ zusammen mit dem Stickstoffatom, an welches sie gebunden sind, für Pyrrolidin oder Piperidin stehen; pharmazeutisch annehmbare Salze davon; und Niederalkylenolether und Niederalkanoylenolester von Verbindungen der Formel I, worin $R^4$ Hydroxy bedeutet, oder Tautomere von diesen Verbindungen, worin $R^4$ Hydroxy oder $NHR^5$ bedeutet.

2. Verbindungen der Formel I gemäss Anspruch 1, worin Ar für Phenyl steht oder für Phenyl, welches mono-oder disubstituiert ist durch einen Substituenten ausgewählt aus der Gruppe bestehend aus Niederalkyl, Niederalkoxy, Niederalkylthio, Niederalkylsulfinyl, Niederalkylsulfonyl, Halogen, Cyan, Carboxy, Niederalkoxycarbonyl, Carbamoyl und Trifluormethyl; oder worin Ar 1-oder 2-Naphthyl bedeutet; oder worin Ar einen fünfgliedrigen ungesättigten heterocyclischen Rest bedeutet, der über Kohlenstoff gebunden ist und ein Heteroatom ausgewählt unter Schwefel, Sauerstoff und unsubstituiertem oder durch Niederalkyl substituiertem Aminostickstoff enthält, oder einen solchen Rest, der zwei Heteroatome, ausgewählt unter einem Iminostickstoff und einem Vertreter aus der Gruppe unsubstituierter oder durch Niederalkyl substituierter Aminostickstoff, Schwefel und Sauerstoff, enthält; oder worin Ar einen sechgliedrigen ungesättigten heterocyclischen Rest bedeutet, der über Kohlenstoff gebunden ist und ein oder zwei Stickstoffatome enthält; oder worin Ar einen bicyclischen benzokondensierten fünfgliedrigen ungesättigten heterocyclischen Rest bedeutet, der über Kohlenstoff gebunden ist und ein Heteroatom, ausgewählt unter Schwefel, Sauerstoff und unsubstituiertem oder durch Niederalkyl substituiertem Aminostickstoff, enthält; oder worin Ar einen bicyclischen bezokondensierten fünfgliedrigen ungesättigten heterocyclischen Rest bedeutet, der über Kohlenstoff gebunden ist und zwei Heteroatome, ausgewählt unter einem Iminostickstoff und einem Vertreter aus der Gruppe unsubstituierter oder durch Niederalkyl substituierter Aminostickstoff, Sauerstoff und Schwefel, enthält; oder worin Ar einen bicyclischen benzokondensierten sechsgliedrigen ungesättigten heterocyclischen Rest bedeutet, der über Kohlenstoff gebunden ist und ein oder zwei Stickstoffatome enthält; oder worin Ar einen dieser heterocyclischen Reste bedeutet, die an Kohlenstoffatomen durch Niederalkoxy, Niederalkyl oder

Halogen mono- oder disubstituiert sind; worin n, R und $R^1$-$R^6$ wie in Ansprch 1 definiert sind; pharmazeutisch annehmbare Salze davon; die entsprechenden Tautomere der Formel Ia von Verbindungen der Formel I, worin $R^4$ für Hydroxy oder $NHR^5$ steht; Niederalkylenolether davon und Niederalkanoylenolester von Verbindungen, worin $R^4$ Hydroxy bedeutet.

3. Verbindungen der Formel I gemäss Anspruch 1, worin Ar Phenyl bedeutet oder Phenyl, welches mono- oder disubstituiert ist durch einen Rest ausgewählt aus der Gruppe bestehend aus Niederalkyl, Niederalkoxy, Niederalkylthio, Niederalkylsulfinyl, Niederalkylsulfonyl, Halogen, Cyan, Carboxy, Niederalkoxycarbonyl, Carbamoyl und Trifluormethyl; oder worin Ar einen fünfgliedrigen ungesättigten heterocyclischen Rest bedeutet, der über Kohlenstoff gebunden ist und zwei Heteroatome, ausgewählt unter einem Iminostickstoff und einem Vertreter aus der Gruppe unsubstituierter oder durch Niederalkyl substituierter Aminostickstoff, Schwefel und Sauerstoff, enthält; oder worin Ar einen sechsgliedrigen ungesättigten heterocyclischen Rest bedeutet, der über Kohlenstoff gebunden ist und ein oder zwei Stickstoffatome enthält; worin n, R und $R^1$-$R^6$ wie in Anspruch 1 definiert sind; pharmazeutisch annehmbare Salze davon; die entsprechenden Tautomer der Formel Ia von Verbindungen der Formel I, worin $R^4$ für Hydroxy oder $NHR^5$ steht.

4. Verbindungen gemäss Anspruch 1, worin n null bedeutet.

5.. Verbindungen der Formel I gemäss Anspruch 1, oder Tautomere davon, worin Ar Phenyl bedeutet oder Phenyl, welches mono- oder disubstituiert ist durch einen Rest ausgewählt aus der Gruppe bestehend aus Niederalkyl, Niederalkoxy, Niederalkylthio, Niederalkylsulfinyl, Niederalkylsulfonyl, Halogen, Cyan, Carboxy, Niederalkoxycarbonyl, Carbamoyl und Trifluormethyl; oder worin Ar 1- oder 2-Naphthyl bedeutet; oder worin Ar einen heterocyclischen Rest bedeutet, der über Kohlenstoff an das Amid-Stickstoffatom gebunden ist und augewählt ist unter Furyl, Pyrrolyl, Thienyl, Thiazolyl, Oxazolyl, Pyridyl, Pyrimidyl, Pyrazinyl, Benzofuranyl, Indolyl, Isochinolyl, Chinolyl, Imidazolyl, Isoxazolyl, Benzimidazolyl, Benzoxazolyl oder einem dieser heterocyclischen Reste, die an Kohlenstoff durch Niederalkyl, Niederalkoxy oder Halogen mono- oder disubstituiert sind, oder einem dieser unsubstituierten oder substituierten Pyrrolyl-, Indolyl-, Imidazolyl- oder Benzimidazolyl-Reste, die am Stickstoffatom durch Niederalkyl substituiert sind; worin n null bedeutet; $R^4$ Hydroxy bedeutet; R, $R^1$, $R^2$ und $R^3$ wie in Anspruch 2 definiert sind; pharmazeutisch annehmbare Salze davon; Niederalkylenolether davon; und Niederalkanoylenolester davon.

6. Verbindungen der Formel I gemäss Anspruch 1, oder Tautomere davon, worin n null bedeutet; Ar für Phenyl steht oder für Phenyl, welches mono- oder disubstituiert ist durch einen Rest ausgewählt aus der Gruppe bestehend aus Niederalkyl, Niederalkoxy, Niederalkylthio, Niederalkylsulfinyl, Niederalkylsulfonyl, Halogen und Trifluormethyl; oder worin Ar einen heterocyclkischen Rest bedeutet, der über Kohlenstoff an das Amid-Stickstoffatom gebunden ist und ausgewählt ist unter Furyl, Pyrrolyl, thienyl, Thiazolyl, Oxazolyl, Pyridyl, Pyrimidyl, Pyrazinyl, Benzofuranyl, Indolyl, Isochinolyl und Chinolyl, oder einem dieser heterocyclischen Reste, die am Kohlenstoff durch Niederalkyl, Niederalkoxy oder Halogen mono- oder disubstituiert sind, oder einem unsubstituierten oder substituierten Pyrrolyl- oder Indolyl-Rest, welcher am Stickstoff durch Niederalkyl substituiert ist; worin R und $R^1$ unabhängig voneinander Wasserstoff, Niederalkyl, Phenylniederalkyl oder Phenylniederalkyl, welches am Phenylteil durch einen oder zwei Reste, ausgewählt unter Niederalkyl, Niederalkoxy, Niederalkylthio, Niederalkylsulfinyl, Niederalkylsulfonyl, Halogen und Trifluormethyl, substituiert ist, bedeuten; $R^2$ und $R^3$ unabhängig voneinander Wasserstoff, Niederalkyl, Halogen, Trifluormethyl oder Niederalkoxy bedeuten; $R^4$ für Hydroxy steht; pharmazeutisch annehmbare Salze davon, die von pharmazeutisch annehmbaren Basen abgeleitet sind; oder pharmazeutisch annehmbare Säureadditionssalze davon, falls Ar einen basischen heterocyclischen Rest bedeutet; Niederalkylenoletherderivate davon; sowie Niederalkanoylenolesterderivate davon.

7. Verbindungen der Formel I gemäss Anspruch 1, worin n null, eins oder zwei bedeutet; Ar für Phenyl steht oder Phenyl bedeutet, welches mono-oder disubstituiert ist durch einen Rest ausgewählt aus der Gruppe bestehend aus Niederalkyl, Niederalkoxy, Niederalkylthio, Carboxy, Halogen und Trifluormethyl; oder worin Ar Thiazolyl oder Pyridyl bedeutet; worin R und $R^1$ unabhängig voneinander Wasserstoff, Niederalkyl, Phenylniederalkyl oder Phenylniederalkyl, welches im Phenylteil durch einen oder zwei Reste, ausgewählt unter Niederalkyl, Niederalkoxy, Niederalkylthio, Halogen und Trifluormethyl, substituiert ist, bedeuten; $R^2$ und $R^3$ unabhängig voneinander Wasserstoff, Niederalkyl, Halogen, Trifluormethyl oder Niederalkoxy bedeuten; $R^4$ für Hydroxy, Pyrrolidino, Phenylniederalkylamino oder Phenylniederalkylamino, welches im Phenylteil durch Halogen substituiert ist, steht; pharmazeutisch annehmbare Salze davon, die von pharmazeutisch annehmbaren Basen abgeleitet sind; oder pharma zeutisch annehmbare Säureadditionssalze davon, falls Ar einen basischen heterocyclischen Rest bedeutet; Niederalkylenoletherderivate davon; und Niederalkanoylenolesterderivate davon.

8. Verbindung der Formel I gemäss Anspruch 1, oder Tautomere davon, worin n null bedeutet, Ar für Phenyl steht oder für Phenyl, welches durch $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, Halogen oder Trifluormethyl monosubstituiert ist, oder für Phenyl, welches durch Reste ausgewählt unter $C_1$-$C_4$-Alkyl, $C_1$-$C_4$-Alkoxy und Halogen disubstituiert ist; worin R Wasserstoff, $C_1$-$C_4$-Alkyl, Phenyl-$C_1$-$C_4$-alkyl oder Phenyl-$C_1$-$C_4$-alkyl, welches im Phenylteil durch $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, Halogen oder Trifluormethyl monosubstituiert ist, oder Phenyl-$C_1$-$C_4$-alkyl, welches in Phenylteil durch Reste ausgewählt unter $C_1$-$C_4$-Alkyl, $C_1$-$C_4$-Alkoxy und Halogen disubstituiert ist, bedeutet; worin $R^1$ Wasserstoff, $C_1$-$C_4$-Alkyl,

Phenyl-$C_1$-$C_4$-alkyl oder Phenyl-$C_1$-$C_4$-alkyl, welches im Phenylteil durch $C_1$-$C_4$-Alkyl, $C_1$-$C_4$-Alkoxy, $C_1$-$C_4$-Alkyltio, $C_1$-$C_4$-Alkylsulfinyl, $C_1$-$C_4$-Alkylsulfonyl, Halogen oder Trifluormethyl monosubstituiert ist, oder Phenyl-$C_1$-$C_4$-alkyl, welches im Phenylteil durch Reste ausgewählt unter $C_1$-$C_4$-Alkyl, $C_1$-$C_4$-Alkoxy und Halogen disubstituiert ist, bedeutet; worin $R^2$ Wasserstoff, $C_1$-$C_4$-Alkyl, $C_1$-$C_4$-Alkoxy, Halogen oder Trifluormethyl bedeutet; $R^3$ für Wasserstoff, $C_1$-$C_4$-Alkyl, $C_1$-$C_4$-Alkoxy oder Halogen steht; $R^4$ Hydroxy bedeutet; und pharmazeutisch annehmbare Salze abgeleitet von pharmazeutisch annehmbaren Basen.

9. Verbindungen der Formel I gemäss Anspruch 1, oder Tautomere davon, worin n null bedeutet; Ar einen heterocyclischen Rest bedeutet ausgewählt unter 2-Pyridyl und 2-Thiazolyl, oder einen solchen Rest, der an Kohlenstoff durch $C_1$-$C_4$-Alkyl oder Halogen substituiert ist; R Wasserstoff, $C_1$-$C_4$-Alkyl, Phenyl-$C_1$-$C_4$-alkyl oder Phenyl-$C_1$-$C_4$-alkyl, welches im Phenylteil durch $C_1$-$C_4$-Alkyl, $C_1$-$C_4$-Alkoxy, $C_1$-$C_4$-Alkylthio, Halogen oder Trifluormethyl monosubstituiert ist, oder Phenyl-$C_1$-$C_4$-alkyl, welches im Phenylteil durch einen Rest ausgewählt unter $C_1$-$C_4$-Alkyl, $C_1$-$C_4$-Alkoxy und Halogen disubstituiert ist, bedeutet; $R^1$ für Wasserstoff, $C_1$-$C_4$-Alkyl, Phenyl-$C_1$-$C_4$-alkyl, oder Phenyl-$C_1$-$C_4$-alkyl, welches im Phenylteil durch $C_1$-$C_4$-Alkyl, $C_1$-$C_4$-Alkoxy, $C_1$-$C_4$-Alkylthio, Halogen oder Trifluormethyl monosubstituiert ist, oder Phenyl-$C_1$-$C_4$-alkyl, welches im Phenylteil durch einen Rest ausgewählt unter $C_1$-$C_4$-Alkyl, $C_1$-$C_4$-Alkoxy und Halogen disubstituiert ist, bedeutet; worin $R^2$ Wasserstoff, $C_1$-$C_4$-Alkyl, $C_1$-$C_4$-Alkoxy, Halogen oder Trifluormethyl bedeutet; $R^3$ für Wasserstoff, $C_1$-$C_4$-Alkyl, $C_1$-$C_4$-Alkoxy oder Halogen steht; $R^4$ Hydroxy bedeutet; und pharmazeutisch annehmbare Salze davon, abgeleitet von pharmazeutisch annehmbaren Basen, oder pharmazeutisch annehmbare Säureadditionssalze davon.

10. Verbindungen gemäss Anspruch 1 der Formel II,

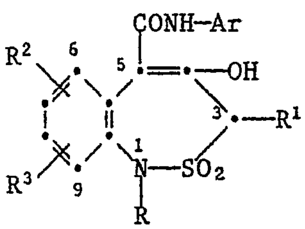

(II)

oder Tautomere davon, worin Ar für Phenyl steht oder Phenyl bedeutet, welches durch $C_1$-$C_4$-Alkyl, $C_1$-$C_4$-Alkoxy, $C_1$-$C_4$-Alkylthio, Halogen oder Trifluormethyl monosubstituiert ist; R $C_1$-$C_4$-Alkyl, Phenyl-$C_1$-$C_4$-alkyl oder Phenyl-$C_1$-$C_4$-alkyl, welches im Phenylteil durch Halogen oder Trifluormethyl monosubstituiert ist, bedeutet; $R^1$ für Wasserstoff, $C_1$-$C_4$-Alkyl oder Phenyl-$C_1$-$C_4$-alkyl steht; $R^2$ Wasserstoff, Halogen, Trifluormethyl oder $C_1$-$C_4$-Alkyl bedeutet; $R^3$ Wasserstoff bedeutet; und pharmazeutisch annehmbare Salze abgeleitet von pharmazeutisch annehmbaren Basen.

11. Verbindungen gemäss Anspruch 10 der Formel II oder Tautomere davon, worin Ar Phenyl bedeutet oder Phenyl, welches durch Methyl, Methylthio, Methoxy, Chlor, Fluor oder Trifluormethyl monosubstituiert ist, R Methyl, Benzyl oder Benzyl, welches im Phenylteil durch Halogen oder Trifluormethyl substituiert ist, bedeutet; $R^1$, $R^2$ und $R^3$ Wasserstoff bedeuten; und pharmazeutisch annehmbare Salze abgeleitet von pharmazeutisch annehmbaren Basen.

12. N-(4-Chlorphenyl)-1-(4-chlorphenylmethyl)-1,3-dihydro-4-hydroxy-2,1-benzothiazepin-5-carboxamid-2,2-dioxid oder ein pharmazeutisch annehmbares Salz davon.

13. N-(4-Chlorphenyl)-1-(4-chlorphenylmethyl)-3-(phenylmethyl)-1,3-dihydro-4-hydroxy-2,1-benzothiazepin-5-carboxamid-2,2-dioxid oder ein pharmazeutisch annehmbares Salz davon.

14. Pharmazeutische Präparate enthaltend eine der in den Ansprüchen 1, 2, 4 bis 6 oder 8 bis 13 beanspruchten Verbindungen.

15. Pharmazeutische Präparate enthaltend eine der in den Ansprüchen 3 oder 7 beanspruchten Verbindungen.

16. Pharmazeutische Präparate enthaltend Verbindungen der Ansprüche 1, 2, 4 bis 6 oder 8 bis 13 zusammen mit einem pharmazeutisch geeigneten Trägermaterial.

17. Pharmazeutische Präparate enthaltend Verbindungen der Ansprüche 3 oder 7 zusammen mit einem pharmazeutisch geeigneten Trägermaterial.

18. Die in den Ansprüchen 1, 2, 4 bis 6 oder 8 bis 13 genannten Verbindungen zur Anwendung in einem Verfahren zur therapeutischen Behandlung des menschlichen oder tierischen Körpers.

19. Die in den Ansprüchen 3 oder 7 genannten Verbindungen zur Anwendung in einem Verfahren zur therapeutischen Behandlung des menschlichen oder tierischen Körpers.

20. Die in den Ansprüchen 1, 2, 4 bis 6 oder 8 bis 13 genannten Verbindungen als Antiinflammatorika.

21. Die in den Ansprüchen 3 oder 7 genannten Verbindungen als ]Antiinflammatorika.

22. Verwendung der in den Ansprüchen 1, 2, 4 bis 6 oder 8 bis 13 genannten Verbindungen zur Herstellung von pharmazeutischen Präparaten.

23. Verwendung der in den Ansprüchen 3 oder 7 genannten Verbindungen zur Herstellung von pharmazeutischen Präparaten.

24. Verwendung der in den Ansprüchen 1, 2, 4 bis 6 oder 8 bis 13 genannten Verbindungen zur

Herstellung von pharmazeutischen Präparaten zur Anwendung als Antiinflammatorika.

25. Verwendung der in den Ansprüchen 3 oder 7 genannten Verbindungen zur Herstellung von pharmazeutischen Präparaten zur Anwendung als Antiinflammatorika.

26. Verfahren zur Herstellung von pharmazentischen Präparaten, dadurch gekennzeichnet, dass eine in den Ansprüchen 1, 2, 4 bis 6 oder 8 bis 13 beanspruchte Verbindung mit einem pharmazentisch geeigneten Trägermaterial aufgearbeitet wird.

27. Verfahren zur Herstellung von pharmazeutischen Präparaten dadurch gekennzeichnet, dass eine in den Ansprüchen 3 oder 7 beanspruchte Verbindung mit einem pharmazeutisch geeigneten Trägermaterial aufgearbeitet wird.

28. Verfahren zur Herstellung von den in Anspruch 1 genannten Verbindungen der Formel I, in der alle Symbole die in Anspruch 1 angegebenen Bedeutungen haben, und deren Salzen, dadurch gekennzeichnet, dass man

a) ein Keton der Formel III,

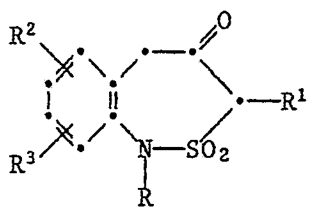

(III)

worin R, $R^1$, $R^2$ und $R^3$ die vorstehend angegebenen Bedeutungen aufweisen, oder ein Enaminderivat der Formel IIIa,

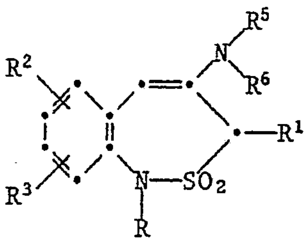

(IIIa)

worin R, $R^1$, $R^2$, $R^3$, und n die vorstehend angegebenen Bedeutungen aufweisen, und $R^5$ und $R^6$ Niederalkyl bedeuten, oder $R^5$ und $R^6$ zusammen mit dem Stickstoffatom, an das sie gebunden sind, Pyrrolidin oder Piperidin bedeuten, mit einer Verbindung der Formel IV,

$Ar\text{-}(CH_2)_n\text{-}N{=}C{=}O$ (IV)

worin Ar und n die vorstehend angegebenen Bedeutungen aufweisen, kondensiert, oder

b) ein reactionsfähiges funktionelles Derivat einer Carbonsäure der Fomel V,

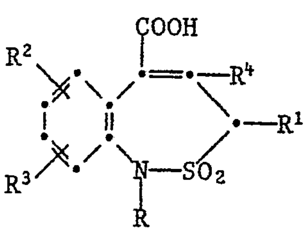

(V)

worin R, $R^1$, $R^2$, $R^3$ und $R^4$ die vorstehend angegebenen Bedeutungen aufweisen, mit einem Amin der Formel VI,

$Ar\text{-}(CH_2)_n\text{-}NH_2$ (VI)

worin Ar und n die vorstehend angegebenen Bedeutungen aufweisen, kondensiert, oder

c) für Verbindungen der Formel I, worin $R^4$ Hydroxy bedeutet, ein reaktionsfähiges funktionelles Derivat einer Carbonsäure der Formel VII,

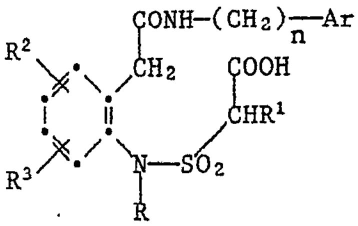

(VIII)

worin R, $R^1$, $R^2$, $R^3$, n und Ar die vorstehend angegebenen Bedeutungen aufweisen, cyclisiert, oder

d) für Verbindungen der Formel I, worin $R^4$ Hydroxy bedeutet, ein reaktionsfähiges funktionelles Derivat einer Carbonsäure der Formel VIII,

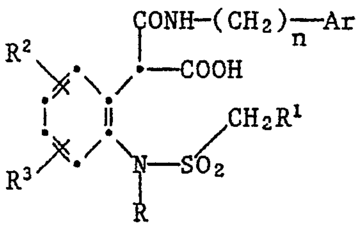

(VII)

worin R, $R^1$, $R^2$, $R^3$, n und Ar die vorstehend angegebenen Bedeutungen aufweisen, cyclisiert, oder

e) für Verbindungen der Formel I, worin $R^4$ Hydroxy bedeutet, ein Enamin der Formel IX,

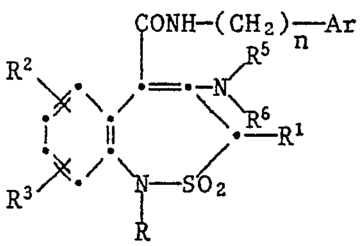

(IX)

worin R, $R^1$, $R^2$, $R^3$, $R^5$, $R^6$, n und Ar die vorstehend angegebenen Bedeutungen aufweisen, hydrolysiert, und in irgendeinem der vorstehenden Verfahren, wenn notwendig, eine erhaltene Verbindung der Formel I in eine andere Verbindung der Formel I überführt, und gegebenenfalls eine erhaltene Verbindung in ein Salz oder ein Salz in ein anderes Salz überführt oder die freie Verbindung aus einem erhaltenen Salz freisetzt.

29. Die nach dem Verfahren des Anspruchs 28 erhältlichen Verbindungen.

Europäisches Patentamt

# EUROPÄISCHER RECHERCHENBERICHT

Nummer der Anmeldung

EP 88 81 0004

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 002 482 (K. THOMAE)<br>* Insgesamt *<br>----- | 1-29 | C 07 D 281/02<br>C 07 D 417/12<br>A 61 K 31/55 |
| | | | **RECHERCHIERTE SACHGEBIETE (Int. Cl.4)** |
| | | | C 07 D 281/00<br>C 07 D 417/00 |

Der vorliegende Recherchenbericht wurde für alle Patentansprüche erstellt

| Recherchenort | Abschlußdatum der Recherche | Prüfer |
|---|---|---|
| DEN HAAG | 14-04-1988 | ALLARD M.S. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)